(19) Europäisches Patentamt
European Patent Office
Office européen des brevets

(11) EP 3 110 450 B1

(12) EUROPEAN PATENT SPECIFICATION

(45) Date of publication and mention of the grant of the patent:
**07.06.2023 Bulletin 2023/23**

(21) Application number: **15755696.0**

(22) Date of filing: **26.02.2015**

(51) International Patent Classification (IPC):
***A61K 47/34*** *(2017.01)* ***A61L 27/52*** *(2006.01)*

(52) Cooperative Patent Classification (CPC):
(C-Sets available)
**A61L 27/52; A61L 27/18; A61L 27/3804; A61L 27/3808; A61L 27/3813; A61L 27/3821; A61L 27/3826; A61L 27/3834; A61L 27/46; A61L 27/54;** A61L 2300/414 (Cont.)

(86) International application number:
**PCT/US2015/017641**

(87) International publication number:
**WO 2015/130878 (03.09.2015 Gazette 2015/35)**

(54) **DEGRADABLE HYDROGEL WITH PREDICTABLE TUNING OF PROPERTIES, AND COMPOSITIONS AND METHODS THEREOF**

ABBAUBARES HYDROGEL MIT VORHERSAGBARER ABSTIMMUNG VON EIGENSCHAFTEN SOWIE ZUSAMMENSETZUNGEN UND VERFAHREN DAFÜR

HYDROGEL DÉGRADABLE AVEC AJUSTEMENT PRÉVISIBLE DES PROPRIÉTÉS, ET COMPOSITIONS ET PROCÉDÉS POUR CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2014 US 201461945108 P**

(43) Date of publication of application:
**04.01.2017 Bulletin 2017/01**

(73) Proprietor: **University of Massachusetts Medical School**
**Boston, MA 02100 (US)**

(72) Inventors:
- **SONG, Jie**
**Shrewsbury, MA 01545 (US)**
- **XU, Jianwen**
**Shrewsbury, MA 01545 (US)**

(74) Representative: **Ter Meer Steinmeister & Partner Patentanwälte mbB**
**Nymphenburger Straße 4**
**80335 München (DE)**

(56) References cited:
**WO-A1-2012/116250 WO-A1-2012/116250**
**US-A1- 2004 161 444 US-A1- 2014 031 285**

- **G. W. ASHLEY ET AL: "Hydrogel drug delivery system with predictable and tunable drug release and degradation rates", PROCEEDINGS NATIONAL ACADEMY OF SCIENCES PNAS, vol. 110, no. 6, 23 January 2013 (2013-01-23), pages 2318-2323, XP055222102, US ISSN: 0027-8424, DOI: 10.1073/pnas.1215498110**
- **JIANWEN XU ET AL: "Cytocompatible Poly(ethylene glycol)-co-polycarbonate Hydrogels Cross-Linked by Copper-Free, Strain-Promoted Click Chemistry", CHEMISTRY - AN ASIAN JOURNAL, vol. 6, no. 10, 4 October 2011 (2011-10-04), pages 2730-2737, XP055154044, ISSN: 1861-4728, DOI: 10.1002/asia.201100411**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **JIANWEN XU ET AL: "Bioorthogonally Cross-Linked Hydrogel Network with Precisely Controlled Disintegration Time over a Broad Range", JOURNAL OF THE AMERICAN CHEMICAL SOCIETY, vol. 136, no. 11, 19 March 2014 (2014-03-19), pages 4105-4108, XP055409973, US ISSN: 0002-7863, DOI: 10.1021/ja4130862**
- **ASHLEY ET AL.: 'Hydrogel drug delivery system with predictable and tunable drug release and degradation rates' PNAS, [Online] vol. 110, no. 6, 05 February 2013, ISSN 0027-8424 pages 2318 - 2323;, XP055222102 ISSN: 0027-8424 Retrieved from the Internet: <URL:http://www.pnas.org/content/11016/2318 . full.pdf>**
- **XU ET AL.: 'Cytocompatible Poly(ethylene glycol)-co-polycarbonate Hydrogels Crosslinked by Copper-free, Strain-promoted ''Click'' Chemistry' CHEMISTRY-AN ASIAN JOURNAL, [Online] vol. 6, no. 10, 04 October 2011, ISSN 1861-4728 pages 2730 - 2737., XP055154044 ISSN: 1861-4728 Retrieved from the Internet: <URL:http://www.ncbi.nlm.nih.gov/pmc/articles/PMC3689886/pdf/nihms440243.pdf>**


(52) Cooperative Patent Classification (CPC): (Cont.)

C-Sets
**A61L 27/18, C08L 71/02;**
**A61L 27/46, C08L 71/02**

## Description

### Technical Field of the Invention

The invention generally relates to hydrogels. More particularly, the invention relates to a novel, versatile degradable hydrogel platform that allows predictable tuning of properties. The bioorthogonally crosslinked hydrogel network affords precisely controlled disintegration profiles tunable over a broad range.

### Background of the Invention

Hydrogel with controlled degradation behaviors are especially useful for a variety of biomedical applications (*e.g.*, drug delivery and tissue regeneration). Numerous degradable hydrogel systems have been reported so far. Control over degradation, however, was often limited to a narrow range and was hard to predict by the chemical formulations of the hydrogel. Moreover, tuning of most of the existing degradable polymers was accompanied by significant changes in other macroscopic properties due to the composition-dependent changes in polymer network structures.

Hydrogels are crosslinked water-swollen polymer networks, which have been exploited for a wide range of applications from superabsorbent materials, contact lenses, sensors, microarrays, to protein and polymer purification. (Alvarez-Lorenzo et al. 2010 J. Drug Deliv. Sci. Tec. 20, 237; Holtz et al. 1997 Nature 389, 829; de Lange et al. 2011 Acs Applied Materials & Interfaces 3, 50; Buhrman et al. 2012 BMC Biotechnology 12, 63.)

Highly desired for advanced biomedical applications, such as guided tissue regeneration and drug delivery, are biocompatible hydrogels with controlled degradation rates and robust physical properties. Hydrogel degradation is a complex process, dictated by not only the chemical composition, but also by the structure of the polymer network.

Limited controls over degradation rate have been realized by either incorporating liable linkages with varying cleavage rates, or altering the polymer network structure containing the same labile linkages (which often causes undesired changes in other macroscopic properties), or a combination of both. (Kharkar et al. 2013 Chem. Soc. Rev. 42, 7335; Peppas et al. 2000 European Journal of Pharmaceutics and Biopharmaceutics 50, 27; Zustiak et al. 2010 Biomacromolecules 11, 1348; Li et al. 2011Macromolecules 44, 3567; Griffin et al. 2012 J. Am. Chem. Soc. 134, 13103; Fairbanks et al. 2011Macromolecules 44, 2444; DeForest et al. 2011 Nature Chemistry 3, 925; Kloxin et al. 2010 Biomaterials 31, 1; Dunn et al. 2012 J. Am. Chem. Soc. 134, 7423; Yang et al. 2014 Journal of Materials Chemistry B, 2, 295; Lutolf et al. 2003 Proc. Natl. Acad. Sci. U. S. A. 100, 5413; Ehrbar et al. 2007 Biomacromolecules 8, 3000.) The concept of tailoring the polarity/charge/structure of neighboring groups to affect the hydrolysis rate of labile linkages has seen some successes in degradable hydrogel designs. (Rydholm et al. 2007 Acta Biomaterialia 3, 449; Jo et al. 2009 Soft Matter 5, 440; Ashley et al. 2013 Proc. Natl. Acad. Sci. U. S. A. 110, 2318.) WO 2012/116250 A 1 relates to functional polymers and hydrogels. More particularly, the document concerns versatile monomers and polymers with well-defined functionalities, e.g. polycarbonates and poly(ester-carbonates), compositions thereof and methods for making and using the same.

It is strongly desired that novel approaches and techniques be developed that provide a versatile degradable hydrogel platform that allow predictable tuning of properties over broad ranges.

### Summary of the Invention

The invention provides a novel approach to hydrogels with predictable degradation, which is useful for many biomedical applications where the timely disintegration of the hydrogel (*e.g.*, drug delivery, guided tissue regeneration) is required. The modular hydrogel platform of the invention allows any biomedical researcher/hospital technician with very basic training to fabricate hydrogels with predictable degradation behavior, with or without the encapsulation of a wide range of bioactive molecules, structural fillers, or cells, under mild physiological conditions by simply mixing a few premade components. Precisely controlling hydrogel degradation over a broad range in a predictable manner is achieved via a simple but versatile hydrogel platform that allows formulation of hydrogels under cytocompatible conditions with predictable disintegration time from about 2 days to 250 days or longer and with comparable macroscopic physical properties.

The modular characteristics in combination with the bioorthogonal crosslinking chemistry, excellent mechanical properties and predictable degradation behaviors have never been realized by any single system. In an exemplary embodiment, the hydrogel platform of the invention is based on a well-defined network formed by two pairs of four-armed poly(ethylene glycol) macromers terminated with azide and dibenzocyclooctyl end groups, respectively, via labile or stable linkages. The high-fidelity bioorthogonal reaction between the symmetric hydrophilic macromers enabled robust crosslinking in water, phosphate buffered saline and cell culture medium to afford tough hydrogels capable of withstanding greater than 90% compressive strain. The strategic placement of labile ester linkages near the crosslinking site within this superhydrophilic network, accomplished by facile adjustments of the ratio of the macromers used, enabled broad

tuning of the hydrogel disintegration rates precisely matching with the theoretical predictions based on a first-order linkage cleavage kinetics.

In one aspect, the invention generally relates to a degradable hydrogel having a controllable and predictable gelling kinetics and/or disintegration profile, comprising a bioorthogonally crosslinked network of a first set of macromers and a second set of macromers, wherein the first set of macromers comprises

a macromer (i) having the structural formula of:

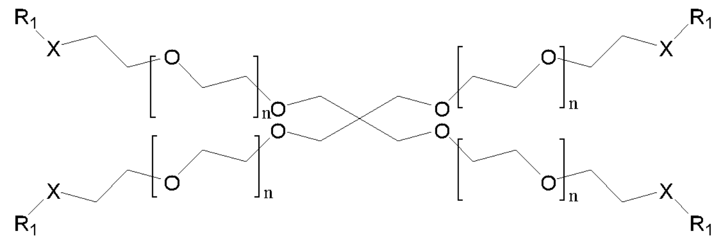

wherein
$R_1$ is a group comprising $-N_3$,
X is an ester group, and
each n is independently an integer from 1 to about 400; and
a macromer (ii) having the structural formula of:

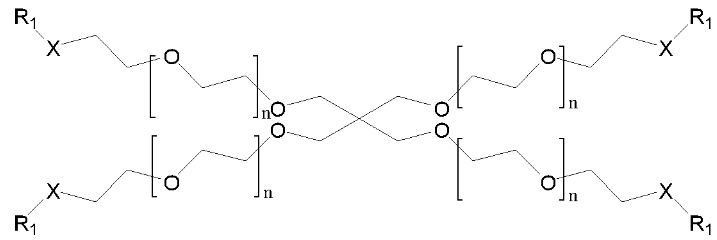

wherein
$R_1$ is a group comprising $-N_3$,
X is empty, and
each n is independently an integer from 1 to about 400; and
the second set of macromers have the structural formula of:

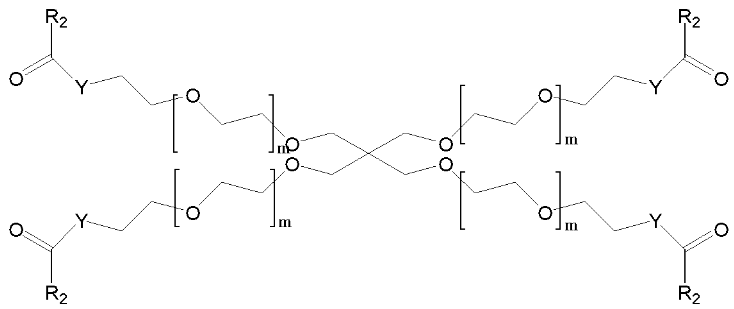

wherein
$R_2$ is a group comprising a cyclic or acylic alkyne group,
Y is NH, O, or empty, and
each m is independently an integer from 1 to about 400,
wherein
the first and second reactive end groups are joined via click chemistry to form a crosslinked network having a controllable and predictable gelling kinetics and/or disintegration profile, and the one or more labile and/or stable linkage(s) are configured within the crosslinked network so as to provide a controllable and predictable gelling kinetics and/or disintegration profile of the hydrogel. The first set of macromers comprises one or more first reactive

end groups, and one or more labile and/or a stable linkage(s). The second set of macromers comprises one or more second reactive end groups, and one or more labile and/or a stable linkage(s). The first and second reactive end groups are bioorthogonally joined via click chemistry to form a crosslinked network having a controllable and predictable gelling kinetics. The one or more labile and/or stable linkage(s) are configured within the crosslinked network so as to provide a controllable and predictable gelling kinetics of the hydrogel.

In another aspect, the invention generally relates to a degradable hydrogel having a controllable and predictable gelling kinetics and/or disintegration profile, comprising a bioorthogonally crosslinked network of a first set of macromers and a second set of macromers, wherein the first set of macromers have the structural formula of:

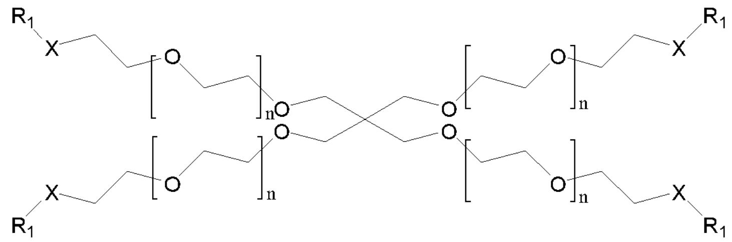

wherein

$R_1$ is a group comprising $-N_3$,
X is an ester group or a carbonate group or is empty, and
each n is independently an integer from 1 to about 400; and
the second set of macromers comprises:

a macromer (iii) having the structural formula of:

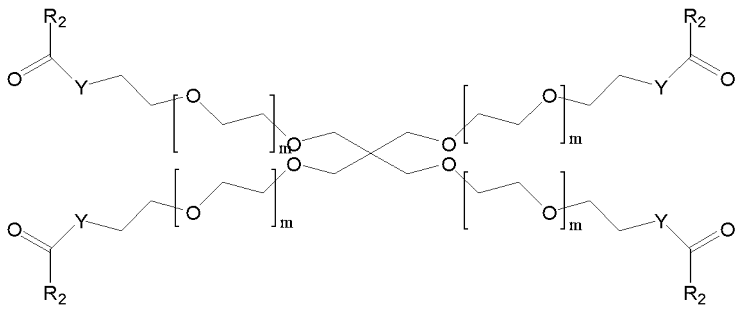

wherein
$R_2$ is a group comprising a cyclic or acylic alkyne group,
Y is O, and
each m is independently an integer from 1 to about 400; and a macromer (iv) having the structural formula of:

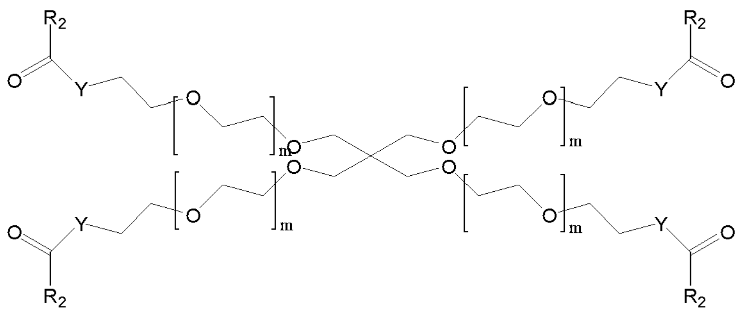

wherein
$R_2$ is a group comprising a cyclic or acylic alkyne group,
Y is NH or empty, and

each m is independently an integer from 1 to about 400,
wherein the first and second reactive end groups are joined via click chemistry to form a degradable crosslinked network having a controllable and predictable gelling kinetics and/or disintegration profile, and
the one or more labile and/or stable linkage(s) are configured within the crosslinked network so as to provide a controllable and predictable gelling kinetics and/or disintegration profile of the hydrogel. The first set of macromers comprises one or more first reactive end groups, and one or more labile and/or a stable linkage(s). The second set of macromers comprises one or more second reactive end groups, and one or more labile and/or a stable linkage(s). The first and second reactive end groups are bioorthogonally joined via click chemistry to form a crosslinked network having a controllable and predictable disintegration profile. The one or more labile and/or stable linkage(s) are configured within the crosslinked network so as to provide a controllable and predictable disintegration profile of the hydrogel.

In yet another aspect, the invention generally relates to a hydrogel composition (e.g., suitable for use in tissue repair or regeneration) comprising a hydrogel of the invention and a three-dimensional construct of one or more payload materials, wherein the one or more payload materials are selected from proteins, growth factors, cytokines, recombinant proteins and gene vectors or an inorganic material selected from calcium apatites, calcium phosphates, hydroxyapatite, and substituted hydroxyapatite. The cytocompatible hydrogel composition includes a three-dimensional construct of one or more payload materials and a bioorthogonally crosslinked network of a first set of macromers and a second set of macromers. The first set of macromers includes one or more first reactive end groups and one or more labile and/or a stable linkage(s). The second set of macromers includes one or more second reactive end groups and one or more labile and/or a stable linkage(s). The one or more payload materials are selected from cells, proteins and minerals. The first and second reactive end groups are bioorthogonally joined via click chemistry to form a crosslinked network having a controllable and predictable disintegration profile. The one or more labile and/or stable linkage(s) are configured within the crosslinked network so as to provide a controllable and predictable disintegration profile of the hydrogel.

In yet another aspect, the invention generally relates to a device or implant comprising a hydrogel composition of the invention.

Described herein is a method for preparing a hydrogel or a composition comprising a hydrogel having a controllable and predictable disintegration profile. The method includes bioorthogonally crosslinking a first set of macromers and a second set of macromers. The first set of macromers includes one or more first reactive end groups and one or more labile and/or a stable linkage(s). The second of macromers includes one or more second reactive end groups and one or more labile and/or a stable linkage(s). The first and second reactive end groups are bioorthogonally joined via click chemistry to form a crosslinked network having a controllable and predictable disintegration profile. The one or more labile and/or stable linkage(s) are configured within the crosslinked network so as to provide a controllable and predictable disintegration profile of the hydrogel.

Described herein is a method for fabricating a hydrogel or a composite thereof, comprising crosslinking a first set of macromers and a second set of macromers. The first set of macromers includes one or more first reactive end groups and one or more labile and/or a stable linkage(s). The second of macromers includes one or more second reactive end groups and one or more labile and/or a stable linkage(s). The first and second reactive end groups are bioorthogonally joined via click chemistry to form a crosslinked network having a controllable and predictable gelling kinetic profile. The one or more labile and/or stable linkage(s) are configured within the crosslinked network so as to provide a controllable and predictable gelling kinetic profile of the hydrogel.

## Brief Description of the Drawings

**FIG. 1.** Schematic of the degradation of an ideally crosslinked and highly swollen homogeneous network containing a single labile linkage precisely positioned between each neighboring netpoints. The cleavage of the labile linkages is assumed to occur independently in a first order kinetics.

**FIG. 2.** Structures and naming of macromers and the orthogonally crosslinked hydrogel (ClickGel) networks.

**FIG. 3.** Four hydrogels crosslinked from different combinations of azide- and DBCO-terminated macromers showing similar macroscopic properties but distinct network disintegration rates. (A) Equilibrium swelling ratio (by weight) of the four hydrogels in PBS (pH=7.4) at 37°C; (B) Stress-strain curves from unconfined compressive testing; (C) Distinct disintegration time of the hydrogels in PBS and in alpha-MEM.

**FIG. 4.** The disintegration time ($t_c$) of a series of hydrogels prepared from 4-armPEG-$N_3$ with varying ratios of 4-armPEG-ester-DBCO and 4-armPEG-amide-DBCO (*r*) predicted by the theoretical model and validated by experimental data. (A) & (B) Theoretical prediction curves of the intact linkage fraction *P vs* time in PBS (pH=7.4) and alpha-MEM, respectively; The red dotted line represents the critical intact linkage fraction of the crosslinked 4-armPEG network ($P_c$ = 1/3), and its crosspoint with each curve indicates the predicted disintegration time for the specific formulation. (C) & (D) Predicted (blue) and experimentally observed (red) hydrogel disintegration time in PBS (pH=7.4) and alpha-MEM, respectively.

**FIG. 5.** The disintegration time ($t_c$) of a series of hydrogels prepared from 4armPEG-amide-DBCO with varying ratios of 4-armPEG-ester-$N_3$ and 4-armPEG-$N_3$ (*r*) predicted by the theoretical model and validated by experimental data. (A) & (B) Theoretical prediction curves of the intact linkage fraction *P vs* time in PBS (pH=7.4) and alpha-MEM, respectively; The red dotted line represents the critical intact linkage fraction of the crosslinked 4-armPEG network ($P_c$ = 1/3), and its crosspoint with each curve indicates the predicted disintegration time for the specific formulation. (C) & (D) Predicted (blue) and experimentally observed (red) hydrogel disintegration time in PBS (pH=7.4) and alpha-MEM, respectively.

**FIG. 6.** $^1$H NMR of dibenzylcyclooctyne-acid (DBCO-acid) in $CDCl_3$.

**FIG. 7.** $^1$H NMR of 4-armPEG20k-OH in $CDCl_3$.

**FIG. 8.** $^1$H NMR of 4-armPEG-$N_3$ in $CDCl_3$.

**FIG. 9.** $^1$H NMR of 4-armPEG-ester-$N_3$ in $CDCl_3$.

**FIG. 10.** $^1$H NMR of 4-armPEG-ester-DBCO in $CDCl_3$.

**FIG. 11.** $^1$H NMR of 4-armPEG-amide-DBCO in $CDCl_3$.

**FIG. 12.** $^{13}$C NMR of dibenzylcyclooctyne-acid (DBCO-acid) in $CDCl_3$.

**FIG. 13.** $^{13}$C NMR of 4-armPEG20k-OH in $CDCl_3$.

**FIG. 14.** $^{13}$C NMR of 4-armPEG-$N_3$ in $CDCl_3$.

**FIG. 15.** $^{13}$C NMR of 4-armPEG-ester-$N_3$ in $CDCl_3$.

**FIG. 16.** $^{13}$C NMR of 4-armPEG-ester-DBCO in $CDCl_3$.

**FIG. 17.** $^{13}$C NMR of 4-armPEG-amide-DBCO in $CDCl_3$.

**FIG. 18.** Nearly complete conversion of reactive functional groups confirmed by spectroscopic measurements. (a) FTIR showing the characteristic peak of azide group at 2100cm$^{-1}$ in azido-containing macromer completely disappeared upon gelling of the hydrogel; and (b) UV-vis measurement showing the characteristic absorption at 307 nm for alkyne group in ClickGel-A with 5 wt% macromer content is lower than that of the 0.1wt% 4-armPEG-amide-DBCO macromer solution.

**FIG. 19.** A demonstration that hydrogels with the same disintegration time but different degradation profiles could be obtained through different formulations enabled by the versatile hydrogel platform. The blue line is the predicted degradation curve for Formulation A, while the black line is the predicted degradation curve for Formulation B. The red dotted line in the plot represents the critical intact linkage fraction ($P_c$) of 1/3 to reach network disintegration.

**FIG. 20.** Examples of a wide range of degradation profiles obtainable with the versatile hydrogel platform based on the simple model involving two formulation-dependent parameters. (A) The prediction curves of intact labile linkage fraction (*P*) over time in PBS and in $\alpha$MEM for formulations with $r^{N3}$ = 0 but varying $r^{DBCO}$; (B) The prediction curves of the intact linkage fraction (*P*) over time in PBS and cell culture media for formulations with $r^{DBCO}$ = 0 but varying $r^{N3}$. The red dotted line in the plots represent the critical intact linkage fraction ($P_c$) of 1/3 for reaching network disintegration.

**FIG. 21.** (A) Time-dependent shear storage moduli (G', red), shear loss moduli (G", blue) and loss tangent (tan delta, black), (B) photo/micrographs of ClickGel-A and HA-ClickGel-A or gelatin MS-ClickGel-A composites. PBS solutions of 5wt% 4armPEG20k-(amide-DBCO)$_4$ and 4armPEG20k-$(N_3)_4$, with/without 10wt% HA or gelatin MS were mixed at rt and loaded on bottom parallel plate of AR-2000 rheometer. Data acquisition started at 2 min (10 rad/s oscillatory angular frequency, 10% stain, 22 °C). Yellow zone indicates gelling timeframe.

**FIG. 22.** (A): Stress-stain curve of ClickGel-A ($20 \times 3 \times 3 mm^3$), 10wt% HA-ClickGel-A composite, and a biphasic construct composed of these compositions (bottom macromers/HA mixture was gelled for 4 min before addition of top phase macromers). All specimens were cured at rt for 4 h before tensile testing on a Q800 DMA (force ramping to 18 N at 1 N/min). *Failure point; (B)-(D): Photographs of strained specimens.

**FIG. 23.** Gelling kinetics of ClickGel can be altered by varying the ratio of azido-macromers with varying neighboring linker to the azido end group mixed with DBCO-terminated macromers. The gelling time, determined from the crosspoint of G' and G" curves plus 2-min for loading mixture to the rheometer, can be tuned from less than 2 min to 5min by increasing the percentage of 4-armPEG-$N_3$ in the total $N_3$-terminated macromers. The dynamic rheology test was performed on an AR-2000 rheometer (TA Instruments) equipped with 8-mm parallel plates and a Peltier heating unit. The gelling process of the various formulations and the evolution of the shear modulus of the hydrogels were studied by oscillatory time sweep rheology experiments at 37 °C. Aqueous solutions of azido-terminated and DBCO-terminated 4-arm PEG macromers (5 w/w%) in PBS (pH =7.4) with 1: 1 molar ratio of the azide groups to the DBCO groups were loaded on the bottom plate sequentially and mixed by pipette. The experiment and data collection were initiated 2 minutes after mixing to ensure consistency among various formulations. An oscillatory angular frequency of 10 rad/s and strain of 10% were applied.

**FIG. 24.** Examples of (A) azido- ($N_3$) and (B) alkyne-terminated macromers containing ester linker with varying

lengths/hydrophobicity, or reactivity.

**FIG. 25.** 1H NMR spectra of 4-armPEG-$OCOCH_2N_3$.

## Detailed Description of the Invention

The invention provides a novel, simple and robust strategy for achieving widely tunable and predictable degradation rates within hydrogels with consistent macroscopic properties by strategic placement of liable ester linkages within a well-defined network.

For many practical applications of degradable hydrogels, the following three characteristics should be met: (1) bioorthogonal gelation conditions/mechanisms that allow the hydrogel to form in the presence of bioactive molecules, structural fillers, or live cells; (2) robust macroscopic properties (*e.g.*, mechanical properties, swelling behavior) of the hydrogel; and (3) simple formulation handling characteristics and facile gelation without the need for special training in terms of prosecution and ensuring reproducible results.

Hydrogel with predictable degradation behaviors while meeting these characteristics are highly desired for biomedical applications and are in critical needs. It remains extremely challenging to achieve broadly tunable degradation rates for a given polymer network due to the complexity and ill-defined relationship between most polymer network structures and their chemical compositions. This is the case even for chemically simple, widely utilized hydrogel systems such as photopolymerized (meth)acrylated polyethylene glycol (PEG) hydrogels where the poorly-defined networks resulting from uncontrolled radical polymerization led to inconsistent degradation, mechanical and biological properties reported in literature. (Lin et al. 2009 Pharmaceutical Research 26, 631; (21) Nguyen et al. 2012 Biomaterials 33, 6682.)

The invention enables precisely controlling hydrogel degradation over a broad range in a predictable manner. The term "predictable", as used herein, refers to the ability to forecast or predict a particular property (such as disintegration profile) This is achieved via a simple but versatile hydrogel platform that allows formulation of hydrogels with predictable disintegration time from about 2 days to about 250 days or longer yet having comparable macroscopic physical properties.

For example, a well-defined network is formed by two pairs of four-armed poly(ethylene glycol) macromers terminated with azide and dibenzocyclooctyl end groups, respectively, via labile or stable linkages. The high-fidelity bioorthogonal reaction between the symmetric hydrophilic macromers enabled robust crosslinking in water, phosphate buffered saline and cell culture medium to afford tough hydrogels capable of withstanding >90% compressive strain.

Labile ester linkages are strategically placed near the crosslinking site within this superhydrophilic network, which enable broad tuning of the hydrogel disintegration rates precisely matching with the theoretical predictions based on a first-order linkage cleavage kinetics. The ester linkages provide facile adjustments of the ratio of the macromers used.

In a homogenously crosslinked network where all polymer chains are fully tethered with evenly spaced netpoints, the degradation behavior becomes much easier to predict when a single liable linkage is precisely positioned between the neighboring netpoints (**FIG. 1**). The cleavage of the labile linkages within such a network in a highly swollen state can be treated as a pseudo-first-order reaction, where the remaining intact linkage fraction (*P*) over time can be described by a very simple model:

$$P = \frac{[\text{linkage}]_\text{t}}{[\text{linkage}]_0} = \text{e}^{-k_\text{d}\,\text{t}} \qquad \text{Eq. (1)}$$

where $k_\text{d}$ is the rate constant of the labile linkage cleavage, t is the time, $[\text{linkage}]_0$ and $[\text{linkage}]_\text{t}$ are the intact linkage concentration prior to degradation and at time t, respectively. When *P* reaches a critical value $P_c$ where the infinite network no longer exists, the hydrogel disintegrates. This critical value is the same as the critical gelling point during the crosslinking, and is defined by the macromer building block structure and the crosslinking chemistry. Therefore, the disintegration time ($t_c$) for such a degradabe network is determined by $P_c$ and $k_\text{d}$ :

$$t_c = -\frac{\ln P_c}{k_d} \qquad \text{Eq. (2)}$$

Similarly, if two liable linkages with varying cleavage rates are incorporated within such a network, the remaining intact linkage fraction (P) over time can be described as:

$$P = \frac{[\text{linkage}]_\text{t}}{[\text{linkage}]_0} = r\,\text{e}^{-k_\text{d}^\text{f}\text{t}} + (1-\text{r})\text{e}^{-k_\text{d}^\text{s}\text{t}} \qquad \text{Eq. (3)}$$

where r is the percentage of the faster degrading labile linkage among the total labile network linkages, while $k_d^f$ and $k_d^s$ are the cleavage rate constant of the faster and slower degrading labile linkages, respectively. The network disintegration time will thus be determined by 3 intrinsic parameters, $P_c$, $k_d^f$ and $k_d^s$ and 1 formulation parameter, *r*. By simply changing the formulation ratio r, the disintegration time could be tuned anywhere between $-\frac{\ln P_c}{k_d^f}$ and $-\frac{\ln P_c}{k_d^s}$. This concept can be extended to incorporate multiple liable linkages with varying susceptibility to cleavage to provide even more flexibility tuning of the network disintegration rate.

To test this strategy, four-armed poly(ethylene glycol) with $M_n$ of 20,000 g/mol (4-armPEG) were chosen as the base macromer structure due to its well-defined symmetric structure, high hydrophilicity and commercial availability, and strain-promoted azide-alkyne cycloaddition (SPAAC) as the crosslinking chemistry due to its and high reactivity and established bioorthogonality (tolerance to biological species) under physiological conditions (**FIG. 2**). (Agard et al. 2004 J. Am. Chem. Soc. 126, 15046; Xu et al. 2011 Chemistry-an Asian Journal 6, 2730.)

First synthesized were two groups of macromers, with azide ($N_3$) and dibenzocyclooctyl (DBCO) end groups attached to the 4-armPEG via a labile ester or stable (*e.g.*, amide) linkages, respectively. Nearly complete end-group functionalization was accomplished as confirmed by $^1$H and $^{13}$C NMR. **(FIGs. 6-17)**.

Four hydrogels (referred to as ClickGel-A, -B, -C and -D) were prepared by combinatorial mixing of one $N_3$- and one DBCO-terminated macromers in equal molar ratio. All formulations gelled in as rapidly as 5 min, and the degree of crosslinking between the $N_3$- and DBCO-terminated 4armPEG macromers was nearly 100% after 20 h as confirmed by the complete conversion of $N_3$ and DBCO end groups into SPAAC crosslinks, as confirmed by FTIR and UV-vis, respectively (**FIG. 18**).

All four hydrogels exhibited comparable equilibrated swelling ratios at around 1.50 (**FIG. 3a**), with ClickGel-A and -C prepared from 4-arm PEG-$N_3$ swelling slightly more than those prepared from 4-armPEG-ester-$N_3$. Unconfined compressive testing (**FIG. 3b**) showed that all four hydrogels withstood up to 90% compressive strain without breaking, exhibiting nearly identical stress-strain curves with the moduli sharply increasing with increasing stains, which is a typical characteristic of ideal elastic networks. ClickGel-B and -D formed from 4-armPEG-ester-$N_3$ (red and blue curves in **FIG. 3b**) showed slightly higher moduli at larger deformations than the hydrogels formed from 4-armPEG-$N_3$, likely due to some degrees of hydrophobic interactions between the esters.

Despite comparable swelling and mechanical properties, the four hydrogels exhibited distinctly different network disintegration rates. In PBS, ClickGel-A was stable for a very long time (>250 days), while the critical disintegration time ($t_c$) for ClickGel-B, -C and -D in PBS were 21, 130 days and 18 days, respectively. Since the comparable macroscopic properties of these hydrogels support similar network structures, the drastic differences in the degradation rate of these hydrogels can be ascribed to the presence and specific positioning of the liable ester linkages within the otherwise identical SPAAC-crosslinked 4-armPEG netowrk. ClickGel-A does not contain any labile linkages, thus was stable over a long period in both PBS and cell culture media containing a rich source of nucleophiles ($\alpha$MEM). Only one type of liable linkage, the ester linkage from 4-armPEG-***ester***-DBCO or 4-armPEG-***ester***-$N_3$, existed in ClickGel-B and -C, making Eq. (1) suitable for describing the degradation kinetics of these two hydrogels.

According to the Flory and Rehner gelation theory on networks formed by step-polymerization, the critical gelling point $P_c$ for an equal molar mixture of mutually reactive 4-arm macromers is 1/3 (see Supporting Information). (Flory 1946 Chem. Rev. 39, 137; Flory et al. 1943 The Journal of Chemical Physics 11, 521.) With the experimentally determined critical gel disintegration time for ClickGel-B and ClickGel-C *(e.g.,* $t_c$ = 21 and 131 days in PBS, respectively, **FIG. 3C**), the apparent cleaveage rate constants for the two liable ester-linkages could thus be calculated by eq. 2, as $k_d^{N3} = 52.3 \times 10^{-3}$ days$^{-1}$ and $k_d^{DBCO} = 8.5 \times 10^{-3}$ days$^{-1}$ in PBS (pH 7.4) respectively. In $\alpha$MEM, ClickGel-B and -C both degraded much more rapidly, but with the same relative rates as observed in PBS, with respective degradation constants of 0.549 and 0.021 days$^{-1}$, respectively. The $k_d$ value for the non-degradable network chain is 0 in both aqueous media.

It is possible to alter the formulation of ClickGel-A and -B, thus the ratio of the non-labile amide-DBCO vs labile ester-DBCO linkages within the crosslinked network, to prepare hydrogels with any disintegration time ranging from 21 days (-ln(1/3)/0.0523) to being non-degradable (infinite degradation time, -ln(1/3)/0) in PBS, or 2 days (-ln(1/3)/0.549) to being non-degradable in $\alpha$MEM, respectively. To test this strategy, a series of hydrogels with potentially changing gel disintegration time was prepared by varying the ratio of 4-armPEG-ester-DBCO and 4-armPEG-amide-DBCO (formulation parameter *r*) mixed with 4-armPEG-$N_3$ while keeping the [DBCO]/[$N_3$] ratio as 1. These hydrogels exhibited similar macroscopic mechanical properties as expected, and their experimentally determined disintegration time in PBS precisely

matched with those theoretically predicated over a wide range of formulation parameters ($r$ = 0 to 1, **FIGs. 4A** and **4C).** The excellent match between experimental and predicted values was also observed in αMEM **(FIGs. 4B** and **4D)** despite the relatively larger standard deviations of the experimental data (likely due to the more complex nucleophile-rich composition of the cell culture media).

These observations validate the strategy that hydrogel degradation can be controlled through strategic placement of liable linkage within an ideally structured homogeneous network and precisely predicted by a simple model. Although the mechanism of the labile linkage cleavage may vary in different medium environment, the modular hydrogel platform and this validated predication model could still guide the formulation of hydrogels to achieve specific disintegration rate, as long as the labile linkage cleavage rate constant can be experimentally derived for the specific medium of interest using a ClickGel containing only the labile linkage of interest (*e.g.*, GlickGel-B or -C, in this case).

The subtle difference in the location of the hydrolytically labile ester linkage in ClickGel-B vs ClickGel-C (on either side of the SPAAC crosslink) resulted in significant difference in their gel disintegration time (**FIG. 3C**). It is not yet clear as to why the ester linkage located on the DBCO side of the SPAAC crosslink is more labile than the one located on the $N_3$ side (which is a topic of ongoing investigations). Using the same strategy outlined above, hydrogels with highly tunable disintegration time ranging from 130 days to infinitely long were prepared by altering the ratio of 4-armPEG-ester-$N_3$ and 4-armPEG-$N_3$ (formulation parameter r) mixed with 4-armPEG-amide-DBCO while keeping the [DBCO]/[$N_3$] ratio as 1. Similarly, the experimentally determined hydrogel disintegration time of these hydrogels agreed well with the predicted values over a wide range of r value (0-1) in both PBS **(FIGs. 5A** and **5C**) and the cell culture media **(FIGs. 5B** and **5D).**

In the two tunable systems described above, the labile ester linkage was strategically positioned near the SPAAC crosslinks to ensure that the degradation process can be viewed as the playback of the crosslinking process in a slow motion. This is an indispensable design element without which the mathematical adoption of the critical gelling point ($P_c$) for the prediction of the critical hydrogel disintegration time would not have been valid.

It should also be noted that the two systems described above not only offer different ranges of possible gel disintegration time (*e.g.*, 21 days and above in PBS for the system described in **FIG. 4** vs 130 days and above for the one described in **FIG. 5),** but also a wide range of degradation rates prior to reaching the network disintegration (slope of the prediction curves). For instance, although it is feasible to formulate a hydrogel with disintegration time longer than 130 days using either system, one could enable much gradual degradation than the other **(FIG. 19).** This may be particularly useful for applications whereas a more gradual loss in mass or mechanical integrity of the network is required. For instance, scaffold-guided tissue regeneration in older or metabolically challenged patients may take longer than in younger/normal patients, thus requiring more extended structural/mechanical support of a resorbable synthetic tissue scaffold.

Unlike ClickGel-B or -C, ClickGel-D possesses labile ester linkages on both sides of the SPAAC crosslinks. Assuming that the cleavage of these linkages proceeds independently from each other, the labile linkage cleavage kinetics in ClickGel-D could be described as:

$$P = \frac{[\mathrm{linkage}]_t}{[\mathrm{linkage}]_0} = e^{-(k_d^{N3} + k_d^{DBCO})t} \qquad \text{Eq. (5)}$$

Applying the $k_d^{N_3}$ and $k_d^{DBCO}$ experimentally determined from ClickGel-B and ClickGel-C, respectively, the disintegration time for ClickGel-D is thus predicted as 18.1 days in PBS or 1.9 days in αMEM. The disintegration time of ClickGel-D in these aqueous media precisely matched with the theoretical prediction (**FIG. 3C**), validating the proposed model.

All scenarios described thus far involve the use of no more than 3 of the 4 designer macromers. When necessary, the use of all 4 macromers could provide an even more versatile platform to formulate hydrogels with far more refined degradation profiles as described by:

$$\frac{[\mathrm{linkage}]_t}{[\mathrm{linkage}]_0} = (1 - r^{N3})(1 - r^{DBCO}) + (1 - r^{N3})\, r^{DBCO}\, e^{-k_d^{DBCO} t} + r^{N3}\,(1 - r^{DBCO})\, e^{-k_d^{N3} t} + r^{N3} r^{DBCO} e^{-(k_d^{DBCO} + k_d^{N3})\, t} \qquad \text{Eq. (6)}$$

where $r^{N3}$ and $r^{DBCO}$ are the ratio of ester-containing macromers in the total azido-terminated and DBCO-terminated

macromers, respectively, $k_d^{N3}$ and $k_d^{DBCC}$ are the cleavage rate constant of the ester linkage positioned on the $N_3$ and DBCO side of the SPAAC crosslinks, respectively. According to Eq. (6), it should be possible to prepare hydrogels with disintegration time longer than 18 days in PBS or 2 days in $\alpha$MEM using this platform by simply changing the formulation parameters $r^{N3}$ and $r^{DBCO}$ (prediction curves selectively shown in **FIG. 20**).

*Preparation of complex multi-phase or gradient ClickGel composites with robust bulk and interfacial properties*

The critical gelling of ClickGel occurred in 1-5 min, and that such a desirable gelling timeframe was maintained upon the incorporation of structural additives (*e.g.*, 10wt% hydroxyapatite or HA, or gelatin microspheres/MS with 5wt% macromers). Despite the increase of viscosity of the mixture (higher loss moduli, blue symbols, **FIG. 21A**), the HA/gelatin MS composites gelled within the same 1-5 min timeframe as ClickGel-A, as revealed by oscillatory time-sweep experiment (yellow band in **FIG. 21A**; note a 2-min delay in data recording). Sharp declines in tan delta (black symbols, **FIG. 21A)** upon gelling to baseline were observed for all formulations, supporting a highly elastic, near-perfect network for both ClickGel-A and its HA- or gelatin MS-composites. This is in contrast with the plasticity commonly observed with imperfect networks containing untethered chains.

The HA or gelatin MS well-dispersed within the ClickGel **(FIG. 21B)** led to an increase in shear modulus of the network by >2-fold (red circles & triangles vs. squares, **FIG. 21A)** and an increase in tensile failure stress by >3-fold, along with improved tensile elasticity **(FIGs. 22A-C).** The residue steady increases in shear modulus between 5 and 20 min **(FIG. 24A)** suggested that some end-groups remained uncrosslinked after initial mixing, making it possible to sequentially delivery additional ClickGel compositions (*e.g.*, via injections for *in vivo* applications) to form multiphasic constructs with robust interfaces strengthened by SPAAC crosslinks formed across adjacent phases. Indeed, biphasic construct containing a HA-ClickGel composite bottom phase and an un-mineralized ClickGel top phase was readily prepared with robust interfacial integration. The interface of the biphasic construct remained intact at tensile failure **(FIG. 22D,** green arrow). The ultimate failure site of the biphasic construct was located within the relatively weaker ClickGel phase **(FIG. 22D,** red arrows), and the failure stress of the biphasic construct is similar to that of the Clickgel **(FIG. 22A).** No leaking of the encapsulated additives was detected over storage in aqueous buffers.

*Fine-tuning gelling kinetics via the manipulation of macromer chemical ,functionalities near the functional end groups*

Controlling the gelling time is critical for translating injectable hydrogel technology into clinical use (*e.g.*, scaffold-assisted tissue repair or cell delivery). Current strategies for controlling gelling time for injectable hydrogels include increasing concentrations of the hydrogel formulations (hydrogel building blocks, crosslinkers, or reactants), catalysts, or temperature, which could elicit cytotoxicity and/or cause significant changes in the properties of the formed hydrogels. Here, a new method was demonstrated for controlling the gelling kinetics of the ClickGel by simply modulating the hydrophobicity of the linker neighboring the reactive end groups or the reactivity of the end groups of the macromer building blocks without significantly affecting the properties. For instance, as shown in **FIG. 23,** by varying the relative ratios of $N_3$-terminated macromers with or without an ester linker (e.g. 4-armPEG-ester-$N_3$ vs. 4-armPEG-$N_3$, **FIG. 23**) mixed with the DBCO-terminated macromer, gelling time varying from 2 to 5 min can be obtained. Similarly, gelling kinetics can also be tuned by fine-tuning the length and hydrophobicity of the ester linkers neighboring $N_3$-terminated **(FIG. 24A)** or alkyne-terminated **(FIG. 4B)** macromers.

Such an approach can enable independent optimization of gelling kinetics without affect other properties of the resulting gel, thus offer unprecedented flexibility for engineering drug delivery vehicles and medical implants.

Thus, the modular hydrogel platform of the invention allows one to fabricate hydrogels with predictable degradation behavior, in the presence of a wide range of bioactive molecules, organic or inorganic structural fillers, or cells, under mild physiological conditions by simply mixing a few premade components. The modular hydrogel platform based on the 2 pairs of well-defined 4-armPEG macromers, the robust and cytocompatible SPAAC crosslinking chemistry, and the strategic positioning of labile ester linkages enables unprecedented, predictive design of hydrogels with consistent macroscopic physical properties yet highly tunable degradation profile over a broad range. This work underscores the importance of network structure on controlling degradation rates. It accomplishes predictive tuning of degradation rates without the need for introducing complex degradable components via tedious multi-step syntheses, which may also results in hard-to-define degradation products.

Thus, in one aspect, the invention generally relates to a degradable hydrogel having a controllable and predictable gelling kinetics, comprising a bioorthogonally crosslinked network of a first set of macromers and a second set of macromers, wherein the first set of macromers comprises a macromer (i) having the structural formula of:

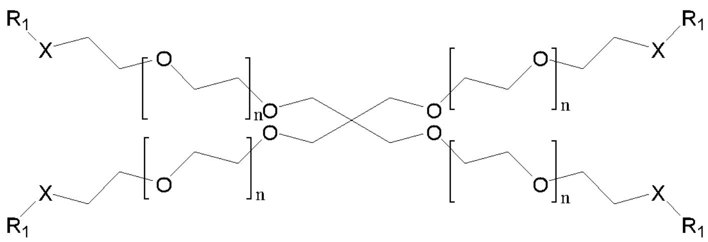

wherein

$R_1$ is a group comprising $-N_3$,
X is an ester group, and
each n is independently an integer from 1 to about 400; and
a macromer (ii) having the structural formula of:

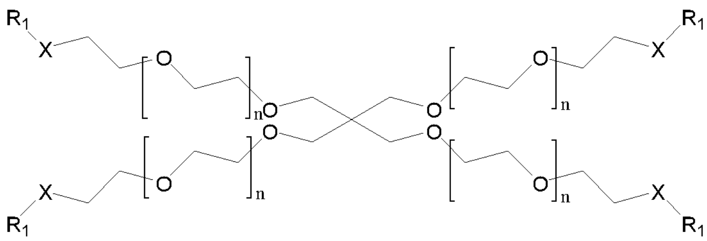

wherein
$R_1$ is a group comprising $-N_3$,
X is empty, and
each n is independently an integer from 1 to about 400; and
the second set of macromers have the structural formula of:

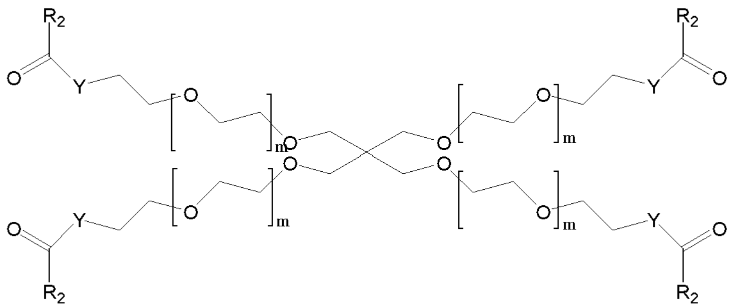

wherein
$R_2$ is a group comprising a cyclic or acylic alkyne group,
Y is NH, O, or empty, and each m is independently an integer from 1 to about 400,
wherein
the first and second reactive end groups are joined via click chemistry to form a crosslinked network having a controllable and predictable gelling kinetics and/or disintegration profile, and the one or more labile and/or stable linkage(s) are configured within the crosslinked network so as to provide a controllable and predictable gelling kinetics and/or disintegration profile of the hydrogel. The first set of macromers comprises one or more first reactive end groups, and one or more labile and/or a stable linkage(s). The second set of macromers comprises one or more second reactive end groups, and one or more labile and/or a stable linkage(s). The first and second reactive end groups are bioorthogonally joined via click chemistry to form a crosslinked network having a controllable and predictable gelling kinetics. The one or more labile and/or stable linkage(s) are configured within the crosslinked network so as to provide a controllable and predictable gelling kinetics of the hydrogel.

In another aspect, the invention generally relates to a degradable hydrogel having a controllable and predictable disintegration profile, comprising a bioorthogonally crosslinked network of a first set of macromers and a second set of

macromers, wherein the first set of macromers have the structural formula of:

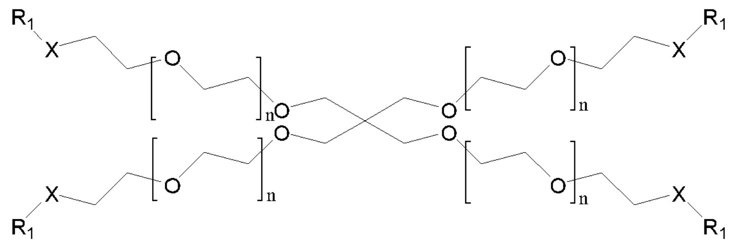

wherein

R1 is a group comprising $-N_3$,
X is an ester group or a carbonate group or is empty, and
each n is independently an integer from 1 to about 400; and
the second set of macromers comprises:

a macromer (iii) having the structural formula of:

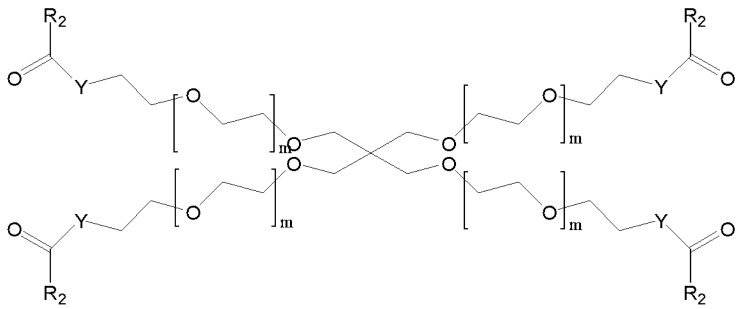

wherein
$R_2$ is a group comprising a cyclic or acylic alkyne group,
Y is O, and
each m is independently an integer from 1 to about 400; and
a macromer (iv) having the structural formula of:

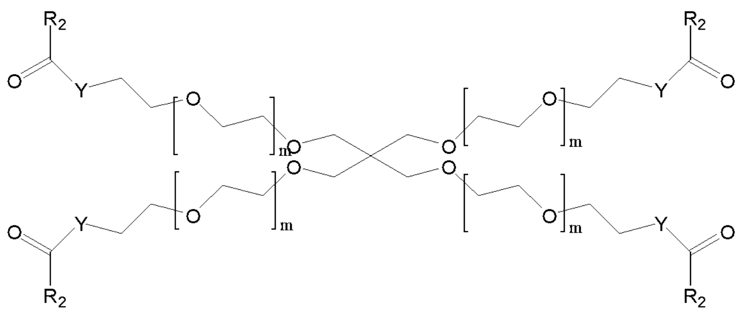

wherein
$R_2$ is a group comprising a cyclic or acylic alkyne group,
Y is NH or empty, and
each m is independently an integer from 1 to about 400,
wherein the first and second reactive end groups are joined via click chemistry to form a degradable crosslinked network having a controllable and predictable gelling kinetics and/or disintegration profile, and
the one or more labile and/or stable linkage(s) are configured within the crosslinked network so as to provide a controllable and predictable gelling kinetics and/or disintegration profile of the hydrogel. The first set of macromers comprises one or more first reactive end groups, and one or more labile and/or a stable linkage(s). The second set of macromers comprises one or more second reactive end groups, and one or more labile and/or

a stable linkage(s). The first and second reactive end groups are bioorthogonally joined via click chemistry to form a crosslinked network having a controllable and predictable disintegration profile. The one or more labile and/or stable linkage(s) are configured within the crosslinked network so as to provide a controllable and predictable disintegration profile of the hydrogel.

In yet another aspect, the invention generally relates to a hydrogel composition (e.g., suitable for use in tissue repair or regeneration) comprising a hydrogel of the invention and a three-dimensional construct of one or more payload materials, wherein the one or more payload materials are selected from proteins, growth factors, cytokines, recombinant proteins and gene vectors or an inorganic material selected from calcium apatites, calcium phosphates, hydroxyapatite, and substituted hydroxyapatites. The cytocompatible hydrogel composition includes a three-dimensional construct of one or more payload materials and a bioorthogonally crosslinked network of a first set of macromers and a second set of macromers. The first set of macromers includes one or more first reactive end groups and one or more labile and/or a stable linkage(s). The second set of macromers includes one or more second reactive end groups and one or more labile and/or a stable linkage(s). The one or more payload materials are selected from cells, proteins and minerals. The first and second reactive end groups are bioorthogonally joined via click chemistry to form a crosslinked network having a controllable and predictable disintegration profile. The one or more labile and/or stable linkage(s) are configured within the crosslinked network so as to provide a controllable and predictable disintegration profile of the hydrogel.

In yet another aspect, the invention generally relates to a device or implant comprising a hydrogel composition of the invention.

Described herein is a method for preparing a hydrogel or a composition comprising a hydrogel having a controllable and predictable disintegration profile. The method includes bioorthogonally crosslinking a first set of macromers and a second set of macromers. The first set of macromers includes one or more first reactive end groups and one or more labile and/or a stable linkage(s). The second of macromers includes one or more second reactive end groups and one or more labile and/or a stable linkage(s). The first and second reactive end groups are bioorthogonally joined via click chemistry to form a crosslinked network having a controllable and predictable disintegration profile. The one or more labile and/or stable linkage(s) are configured within the crosslinked network so as to provide a controllable and predictable disintegration profile of the hydrogel.

Described herein is a method for fabricating a hydrogel or a composite thereof, comprising crosslinking a first set of macromers and a second set of macromers. The first set of macromers includes one or more first reactive end groups and one or more labile and/or a stable linkage(s). The second of macromers includes one or more second reactive end groups and one or more labile and/or a stable linkage(s). The first and second reactive end groups are bioorthogonally joined via click chemistry to form a crosslinked network having a controllable and predictable gelling kinetic profile. The one or more labile and/or stable linkage(s) are configured within the crosslinked network so as to provide a controllable and predictable gelling kinetic profile of the hydrogel.

In certain preferred embodiments, the first set of macromers are poly(ethylene glycol) macromers with a first reactive end groups and the second set of macromers are poly(ethylene glycol) macromers with a second reactive end groups.

In certain preferred embodiments, the first set of macromers comprise four first reactive end groups and the second set of macromers comprise four second reactive end groups. In certain more preferred embodiments, four first reactive end groups are terminal azide groups, and four second reactive end groups are terminal alkyne groups.

In certain preferred embodiments, the first set of macromers has the structural formula of:

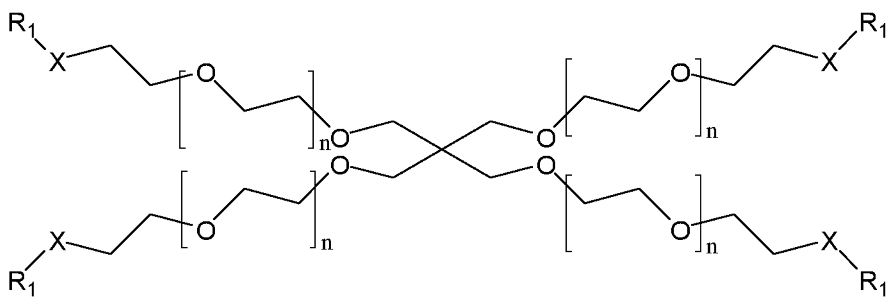

wherein $R_1$ is a group comprising $-N_3$, X is selected from single bond *(i.e.,* "absent or empty", the two adjacent atoms join together through a single C-C bond), ester and carbonate groups, and each n is independently an integer from 1 to about 400 *(e.g.,* from 1 to about 300, from 1 to about 200, from 1 to about 100, from 1 to about 50, from 1 to about 30, from 1 to about 20, from 4 to about 400, from 50 to about 400, from 100 to about 400). The second set of macromers has the structural formula of:

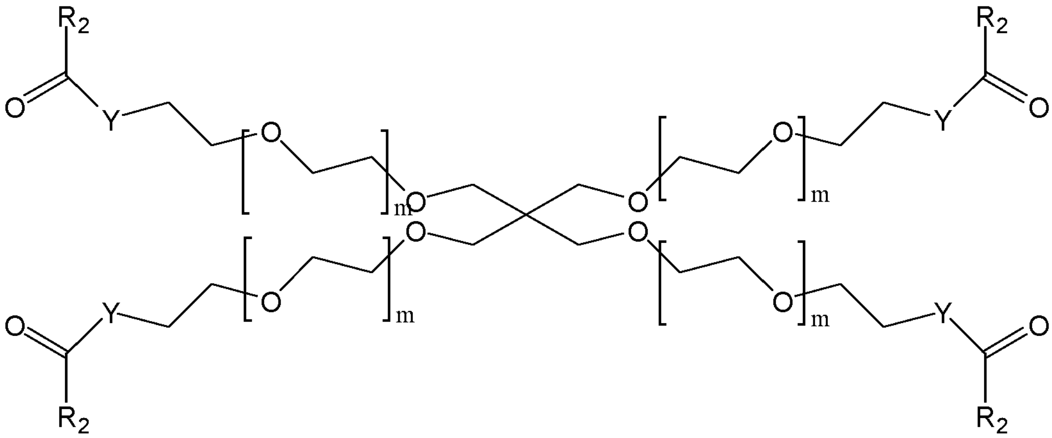

In certain preferred embodiments, $R_2$ is

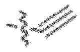

or a group comprising a cyclic or acyclic alkyne group, Y is selected from single bond, -NH- and -O- groups, and each m is independently an integer from 1 to about 400 *(e.g.,* from 1 to about 300, from 1 to about 200, from 1 to about 100, from 1 to about 50, from 1 to about 30, from 1 to about 20, from 4 to about 400, from 50 to about 400, from 100 to about 400).

In certain preferred embodiments, $R_2$ is

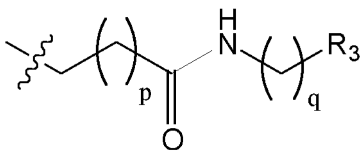

wherein $R_3$ is a group comprising a group comprising a cyclic or acyclic alkyne group, each of p and q is an integer from about 1 to about 6 *(e.g.,* 1, 2, 3, 4, 5, 6). $R_2$ (or $R_3$) may be any suitable group, for example, a group comprising a cyclic alkyne group such as dibenzylcyclooctyne (DBCO) group or an acyclic alkyne group. Other exemplary cyclic alkyne groups include groups such as dibenzocyclooctyne-amine, dibenzocyclooctyne-N-hydroxysuccinimidyl ester, (1R,8S,9s)-bicyclo[6.1.0]non-4-yn-9-ylmethanol, (1R,8S,9s)-bicyclo[6.1.0]non-4-yn-9-ylmethyl N-succinimidyl carbonate, and dibenzocyclooctyne-maleimide.

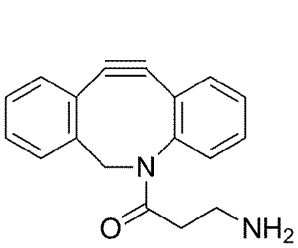

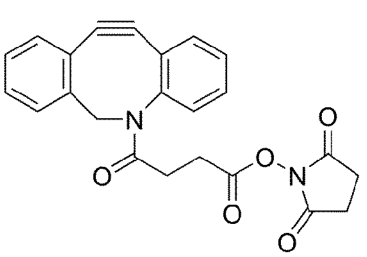

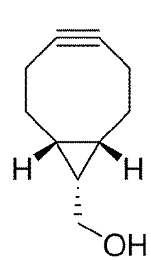

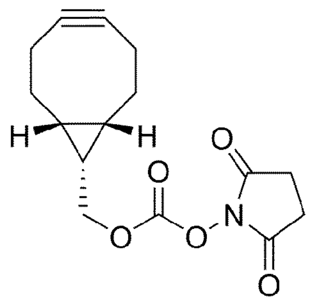

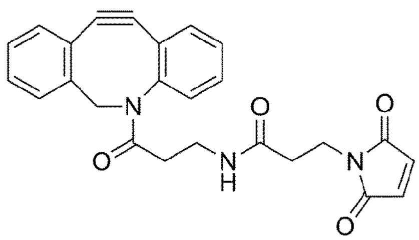

Additional compounds and groups that may be utilized to effect click chemistry can be found in general chemistry literature such as http://www.sigmaaldrich.com/chemistry/chemistry-produtcs.html?TablePage=111698250.

The hydrogel composition of invention may be fine-tuned to achieve a disintegration rate from about 2 days to about 250 days (*e.g.*, from about 2 days to about 200 days, from about 2 days to about 150 days, from about 2 days to about 100 days, from about 2 days to about 50 days, from about 2 days to about 30 days, from about 2 days to about 20 days, from about 2 days to about 10 days, from about 10 days to about 250 days from about 20 days to about 250 days from about 30 days to about 250 days, from about 50 days to about 250 days, from about 100 days to about 250 days) in non-enzymatic aqueous medium.

The bioorthogonally crosslinking may be accomplished via any suitable reactions, for example, the first macromer and the second macromer may be crosslinked via copper-free, strain-promoted azide-alkyne cycloaddition (*e.g.*, when the alkyne group in $R_2$ or $R_3$ is within a cyclic structure) or via copper-catalyzed azide-alkyne cycloaddition (*e.g.*, when the alkyne group in $R_2$ or $R_3$ is acyclic).

In certain preferred embodiments, the gelling kinetic profile is characterized by under 1 min to over 24 h (*e.g.*, about 2 min. about 5 min., about 5 to about 15 min., about 15 min. to about 1 h, about 1 to about 6 h, about 6 to about 12 h, about 12 to about 24).

The payload materials may be suitable materials, including cells, biomolecules, organic and inorganic compounds. In certain preferred embodiments, one or more mammalian cells are selected as the payload, including one or more of bone marrow stromal cells, osteoblasts, chondrocytes, endothelial cells, epithelial cells, embryonic stem cells, mesenchymal stem cells, hematopoietic stem cells, myoblasts, periosteal cells, or cell lines.

The one or more payload materials may include a biomolecule selected from proteins, growth factors, cytokines, recombinant proteins and gene vectors.

In certain preferred embodiments, one or more bone morphogenetic proteins (BMPs) (*e.g.*, BMP1, BMP2, BPM3, BPM4, BMP5, BMP6, BMP7, BMP8a, BMP8b, BMP10, BMP15) and/or transforming growth factor beta (TGF-beta's) (*e.g.*, TGF-beta1, TGF-beta3) are the payload.

The one or more payload materials may include such as inorganic material (*e.g.*, calcium apatites, calcium phosphates, hydroxyapatite, and substituted hydroxyapatites).

**Examples**

The experimental results demonstrate a bioorthogonally crosslinked hydrogel network with precisely controlled disintegration profiles over a broad range

*Materials*

Primary alcohol- and amine-terminated 4-armPEG20k macromers, 4-armPEG20k-OH and 4-armPEG20k-$NH_2$ (Mn = 20,000 g/mol by MALDI-TOF measurements), were obtained from JenKem Technology (Beijing, China), and dried under vacuum in the melt state prior to use. Azadibenzocyclooctyne acid (DBCO-acid) was purchased from Click Chemistry Tools (Macon, GA, USA) and used as received. 4-(Dimethylamino)-pyridinium p-toluenesulfonate and 4-azidobutanoic acid were prepared and purified according to literatures. (Moore et al. 1990 Macromolecules 23, 65; Fraser et al. 2013 Medchemcomm 4, 383.) All other reagents were purchased from Sigma-Aldrich and used as received unless otherwise noted.

*Synthesis of 4-armPEG-ester-$N_3$*

To a 100-mL reaction flask containing 70 mL of chloroform solution of 4-armPEG20k-OH (5.0 g, ~0.25 mmol), was added 4-azidobutanoic acid (0.387 g, ~3.0 mmol), DPTS (149 mg, 0.5 mmol) and DIC (2.524 g, 20.0 mmol). The mixture was reacted at room temperature for 20 h, and the product was purified by dialysis (Spectra/Pro® 7 membrane, MWCO = 2000 Dalton) against 1000 mL of methanol 6 times, concentrated and precipitated in ethyl ether, filtered and dried under vacuum to obtain white powders (~5.1g, 93% yield).

*Synthesis of 4-armPEG-$N_3$*

4-armPEG-$N_3$ was prepared by azidation of 4-armPEG20k-Cl, which was in turn derived from 4armPEG20k-OH. 4-ArmPEG20k-OH (10g, 0.5 mmol) was dissolved in 60 mL of thionyl chloride in a 250-mL reaction flask and refluxed for 20 h. After removing the volatiles by vacuum, the crude product was dissolved in chloroform and washed with saturated brine 6 times. The organic layers were combined and dried over sodium sulfate, concentrated and then precipitated in 600 mL of ethyl ether. The white precipitate was further washed with ethyl ether and hexane 3 times each, and dried under vacuum to give white powders (9.0 g, 90% yield). The as-prepared 4-armPEG20k-Cl (5.0 g, ~0.25 mmol) was

mixed with sodium azide (0.65 g, 10.0 mmol) in 80 mL of DMSO in a 250-mL flask and reacted at 100 °C for 24 h. Ethyl acetate (150 mL) was added to the suspension and stirred at 65 °C for 1 h, and the mixture was then filtered through a column packed with 0.5-cm thick Celite pad. The volatile was removed by vacuum and 150 mL of saturated brine was then added. The solution was extracted with chloroform (150ml) 3 times. The combined chloroform phase was washed with 100-mL saturated brine twice, and then dried over sodium sulfate, concentrated, and precipitated in 1000-mL ethyl ether, filtered and dried under vacuum to obtain white powders (4.5g, 90% yield).

*Synthesis of 4-armPEG-ester-DBCO*

To a 100-mL reaction flask containing 70 mL of chloroform solution of 4-armPEG20k-OH (5.0 g, ~0.25 mmol) was added DBCO-acid (436.8 mg, 1.25 mmol), 4-(dimethylamino)-pyridinium p-toluenesulfonate (DPTS, 149 mg, 0.5 mmol) and N,N-diisopropylcarbodiimide (DIC, 2.524 g, 20.0 mmol). The mixture was reacted at room temperature for 20 h, and then purified by dialysis (Spectra/Pro® 7 membrane, MWCO = 2000 Dalton) against 1000 mL of methanol 6 times, concentrated and precipitated in ethyl ether, filtered and dried under vacuum to obtain pale powders (~5.1g, 95.% yield).

*Synthesis of 4-armPEG-amide-DBCO*

4-armPEG-amide-DBCO was synthesized in a similar manner as the 4-armPEG-ester-DBCO, but using 4-armPEG20k-$NH_2$ as the starting material instead. Pale off white powders (~5.1g, 95% yield) were obtained after similar purification procedures.

*Hydrogel Preparation*

All hydrogels were prepared by mixing equal moles (N3/DBCO = 1) of azide-terminated and DBCO-terminated macromer solutions (5 wt%) phosphate buffered saline (PBS, pH 7.4) For example, to prepare the hydrogel crosslinked from 4-armPEG-$N_3$ and 4-armPEG-amide-DBCO, 51.5 μL of PBS solution of 4-armPEG-$N_3$ was combined with 55.1 μL solution of 4-armPEG-amide-DBCO and thoroughly mixed by vortexing before being poured into a Teflon mold and allowed to gel at room temperature for 20 h until further characterizations or uses. To prepared hydrogel from 3 or more macromers, the azide-terminated macromers and DBCO-terminated macromers were first mixed separately, and then combined before being poured in a Teflon mold for gelling.

*Preparation of functional macromers (4-armPEG-OCOCH2N3, FIG. **24A)***

To a 100-mL reaction flask containing 70 mL of chloroform solution of 4-armPEG20k-OH (5.0 g, ~0.25 mmol), was added 2-azidoacetic acid (0.303 g, ~3.0 mmol), 4-(Dimethylamino)-pyridinium p-toluenesulfonate (73.6 mg, 0.25 mmol) and N, N'-dicyclohexyldicarbodiimide (1.032g, 5.0 mmol). The mixture was reacted at room temperature for 20 h, and the product was filtered through a short column with silica gel and precipitated in ethyl ether. The precipitation was repeated 3 times, and then filtered and dried under vacuum to obtain white powders (-4.6 g, 90% yield). The NMR spectrum of the product is shown in **FIG. 25.**

*Nuclear magnetic resonance (NMR)*

$^1H$ (400 MHz) and $^{13}C$ NMR (100 MHz) spectra were recorded on a Varian INOVA-400 spectrometer in deuterated chloroform ($CDCl_3$, 99.8 atom% D with 0.03% v/v TMS). $^1H$ NMR spectra were obtained with 10-15 mg of samples in 0.7-mL solvent, and $^{13}C$ NMR spectra were obtained with 100-150 mg of samples in 0.7-mL solvent.

*Fourier Transformed Infrared (FTIR) Spectroscopy*

The FTIR spectra were taken on a Nicolet IR 100 spectrometer (Thermo Electron Corporation) with 2-$cm^{-1}$ spectral resolution. Lyophilized maromers and hydrogel samples were mold-pressed with KBr into transparent discs for measurement.

*Ultraviolet visible (UV-vis) spectroscopy*

The UV-vis spectra were taken at 20 °C on a Cary 50 spectrometer (Agilent Technologies) equipped with a peltier thermostat cell holder and a temperature control unit. A Quartz cuvette with 1-cm path length was used. To monitor the conversion of DBCO group during the hydrogel formation, the solution of 5 wt% DBCO-terminated macromer and

the solution of 5 wt% $N_3$-terminated macromers were mixed in-situ in the cuvette at the ratio of DBCO/N3 = 1, and the UV-vis absorption was monitored over time.

*Gelation Time Measurements*

Gelation time of each hydrogel formulation was determined by the inverse tube method. The 5 wt% respective macromer solutions in PBS were mixed in a microfuge tube at rt, vortexed for 20 s, and the gelation was monitored by repeated inversions of the tube. The gelation time was recorded when the hydrogel no longer flowed by gravity.

*Compressive Test*

Unconfined compressive testing was performed on a dynamic mechanical analyzer (DMA800, TA Instruments) at 25 °C. Cylindrical specimens (5 mm × 6 mm, height × diameter) were compressed under the force ramping from 0.01 N to 18 N (the maximum limit of the load cell) at 2 N/min. At least 3 specimens were tested for each sample. The slopes of the stress-versus-strain curves in the linear range of 10-30% strain were used for calculating the elastic moduli.

*Equilibrated Mass Swelling Ratio*

As-prepared crosslinked hydrogel specimens (~50 mg) were placed into 2 mL of 0.1-M PBS (pH 7.4, with 0.02 wt% sodium azide) and incubated at 37 °C. Every 8 hours, the hydrogel specimens were retrieved, removed of excess aqueous buffer by KimWipe, and weighed. After roughly 24 h, when the swelling for the hydrogels became stabilized. The equilibrated mass swelling ratio was determined by the weight of the wet hydrogel at 24 h ($W_t$) versus the weight of the as-prepared specimen ($W_0$) using the following equation:

$$\text{Equilibrated mass swelling ratio } = \frac{W_t}{W_0}$$

These hydrated specimens were subsequently lyophilized, and the weight of the dried sample versus the weight of as-prepared sample was shown to be 5% for all tested hydrogels, which was identical to the macromer content during hydrogel preparation.

*Monitoring of Hydrogel Disintegrations*

The hydrogel degradation in PBS (pH 7.4) or alpha-MEM at 37 °C in a humidified incubator with 5% $CO_2$ was monitored over time. As-prepared crosslinked hydrogel specimens (40-60 mg) were placed in 2 mL of PBS (pH 7.4, supplemented with 0.02 wt% sodium azide) or alpha-MEM (supplemented with 0.02 wt% sodium azide) and incubated at 37°C, with weekly change of fresh PBS or alpha-MEM. To determine the hydrogel gel disintegration time, the integrity of the hydrogel specimen was monitored regularly. The time when the specimen completely disintegrated into the aqueous media was recorded as the gel disintegration time.

*Theoretical Calculation of Critical Gel Disintegration Point*

The hydrolytic degradation of an adequately hydrated, homogeneously crosslinked hydrogel network may be treated as a reverse process of its crosslinking/gelling, in which the polymer network is cut into non-elastic dangling chains until the point where the crosslinked network disintegrates into finite soluble polymer segments. The critical gel disintegration point is the same as the gelation point where an insoluble network forms during crosslinking. The gelation point for a hydrogel formed by step polymerization/crosslinking from two mutually reactive monomers/macromers A and B can be described by Eq. (S1), adapted from the theory by Flory and Rehner: (Flory 1946 Chem. Rev. 39, 137; Flory et al. 1943 The Journal of Chemical Physics 11, 521.)

$$P_c^{step} = \frac{[\text{linkage}]_t}{[\text{linkage}]_0} = \frac{1}{\sqrt{r(f_A - 1)((f_B - 1)}} \qquad \text{Eq. (S1)}$$

where $P_c^{step}$ is the critical fraction of linkages/crosslinks formed between A and B at the gelation point, $f_A$ is the number

of reactive functionality in each monomer/macromer A; $f_B$ is the number of reactive functionality in each monomer/macromer B; and r is the stoichiometric ratio of A to B. Thus, the $P_c$ value for a hydrogel prepared from equal molar mixture ($r$ = 1) of mutually reactive 4-armPEG macromers ($f_A = f_B = 4$) is 1/3.

In this specification and the appended claims, the singular forms "a," "an," and "the" include plural reference, unless the context clearly dictates otherwise.

Unless defined otherwise, all technical and scientific terms used herein have the same meaning as commonly understood by one of ordinary skill in the art. Although any methods and materials similar or equivalent to those described herein can also be used in the practice or testing of the present disclosure, the preferred methods and materials are now described. Methods recited herein may be carried out in any order that is logically possible, in addition to a particular order disclosed.

**Equivalents**

The representative examples are intended to help illustrate the invention, and are not intended to, nor should they be construed to, limit the scope of the invention. The examples contain important additional information, exemplification and guidance that can be adapted to the practice of this invention in its various embodiments and equivalents thereof.

## Claims

**1.** A degradable hydrogel having a controllable and predictable gelling kinetics or disintegration

profile, comprising a bioorthogonally crosslinked network of a first set of macromers and a second set of macromers, wherein
the first set of macromers comprises
a macromer (i) having the structural formula of:

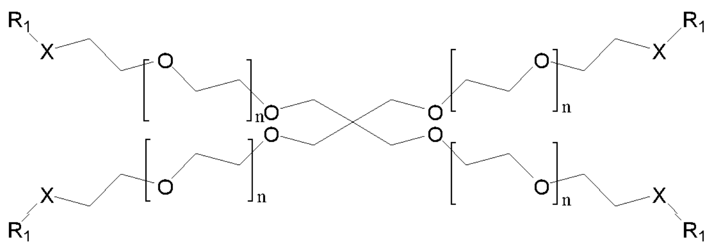

wherein
$R_1$ is a group comprising $-N_3$,
X is an ester group, and
each n is independently an integer from 1 to about 400; and
a macromer (ii) having the structural formula of:

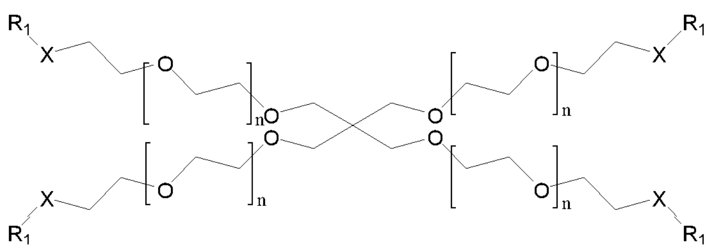

wherein
$R_1$ is a group comprising $-N_3$,
X is empty, and
each n is independently an integer from 1 to about 400; and
the second set of macromers have the structural formula of:

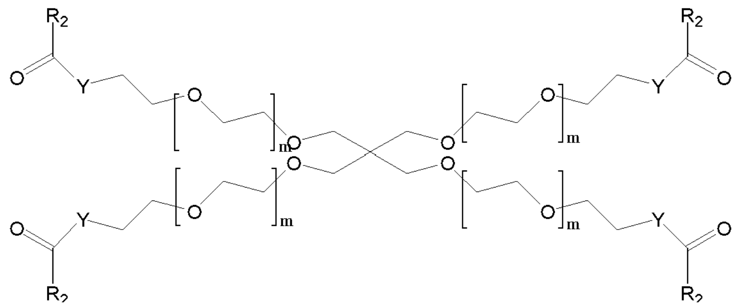

wherein
$R_2$ is a group comprising a cyclic or acylic alkyne group,
Y is NH, O, or empty, and
each m is independently an integer from 1 to about 400,
wherein
the first and second reactive end groups are joined via click chemistry to form a crosslinked network having a controllable and predictable gelling kinetics or disintegration profile, and the one or more labile and/or stable linkages are configured within the crosslinked network so as to provide a controllable and predictable gelling kinetics or disintegration profile of the hydrogel.

**2.** The hydrogel of Claim 1, wherein Y is NH.

**3.** The hydrogel of Claim 1, wherein Y is O.

**4.** The hydrogel of Claim 1, wherein $R_2$ is

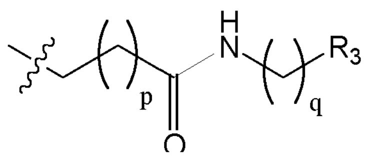

wherein $R_3$ is a group comprising a group comprising a cyclic or acyclic alkyne group, each of p and q is an integer from about 1 to about 6.

**5.** The hydrogel of Claim 4, wherein $R_3$ comprises a group selected from the group consisting of dibenzylcyclooctyne (DBCO), dibenzocyclooctyne-amine, dibenzocyclooctyne-N-hydroxysuccinimidyl ester, (1R,8S,9s)-Bicyclo[6.1.0]non-4-yn-9-ylmethanol, (1R,8S,9s)-bicyclo[6.1.0]non-4-yn-9-ylmethyl N-succinimidyl carbonate, and dibenzocyclooctyne-maleimide groups.

**6.** The hydrogel of Claim 5, wherein crosslinking the first set of macromers and the second set of macromers is performed via copper-free, strain-promoted azide-alkyne cycloaddition or copper-catalyzed azide-alkyne cycloaddition.

**7.** A hydrogel composition, comprising a hydrogel of claim 1 and a three-dimensional construct of one or more payload materials, wherein the one or more payload materials are selected from proteins, growth factors, cytokines, recombinant proteins and gene vectors or an inorganic material selected from calcium apatites, calcium phosphates, hydroxyapatite, and substituted hydroxyapatites.

**8.** A device or implant comprising a hydrogel composition of Claim 7.

**9.** A degradable hydrogel having a controllable and predictable gelling kinetics or disintegration

profile, comprising a bioorthogonally crosslinked network of a first set of macromers and a second set of macromers, wherein
the first set of macromers have the structural formula of:

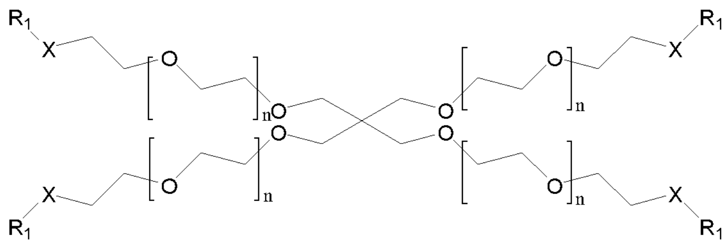

wherein
$R_1$ is a group comprising $-N_3$,
X is an ester group or a carbonate group or is empty, and
each n is independently an integer from 1 to about 400; and
the second set of macromers comprises:

a macromer (iii) having the structural formula of:

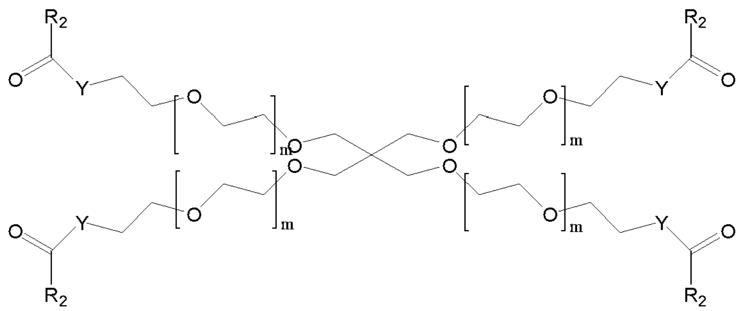

wherein
$R_2$ is a group comprising a cyclic or acylic alkyne group,
Y is O, and
each m is independently an integer from 1 to about 400; and
a macromer (iv) having the structural formula of:

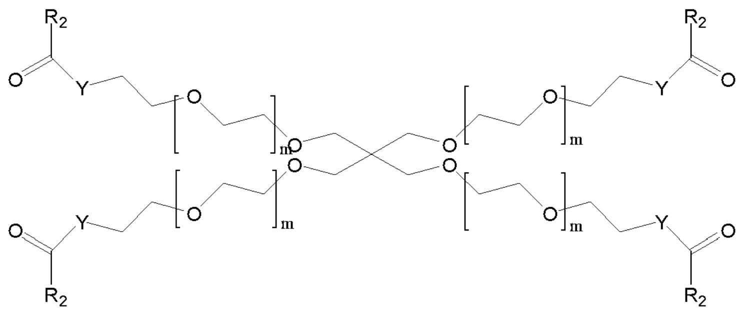

wherein
$R_2$ is a group comprising a cyclic or acylic alkyne group,
Y is NH or empty, and
each m is independently an integer from 1 to about 400,
wherein the first and second reactive end groups are joined via click chemistry to form a degradable crosslinked network having a controllable and predictable gelling kinetics or disintegration profile,
and
the one or more labile and/or stable linkages are configured within the crosslinked network so as to provide a controllable and predictable gelling kinetics or disintegration profile of the hydrogel.

**10.** The hydrogel of Claim 9, wherein $R_2$ is

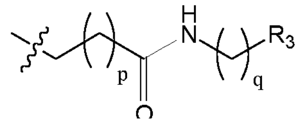

wherein $R_3$ is a group comprising a cyclic or acyclic alkyne group, each of p and q is an integer from about 1 to about 6.

**11.** The hydrogel of Claim 10, wherein $R_3$ comprises a group selected from the group consisting of dibenzylcyclooctyne (DBCO), dibenzocyclooctyne-amine, dibenzocyclooctyne-N-hydroxysuccinimidyl ester, (1R,8S,9s)-Bicyclo[6.1.0] non-4-yn-9-ylmethanol, (1R,8S,9s)-bicyclo[6.1.0]non-4-yn-9-ylmethyl N-succinimidyl carbonate, and dibenzocyclooctyne-maleimide groups.

**12.** The hydrogel of Claim 11, wherein crosslinking the first set of macromers and the second set of macromers is performed via copper-free, strain-promoted azide-alkyne cycloaddition or copper-catalyzed azide-alkyne cycloaddition.

**13.** The hydrogel of Claim 9, wherein X is a carbonate group.

**14.** A hydrogel composition, comprising a hydrogel of claim 9 and a three-dimensional construct of one or more payload materials, wherein the one or more payload materials are selected from proteins, growth factors, cytokines, recombinant proteins and gene vectors or an inorganic material selected from calcium apatites, calcium phosphates, hydroxyapatite, and substituted hydroxyapatites.

**15.** A device or implant comprising a hydrogel composition of claim 14.

**Patentansprüche**

**1.** Abbaubares Hydrogel mit einem steuerbaren und vorhersagbaren Profil der Kinetik der Gelierung oder der Zersetzung, umfassend ein bioorthogonal vernetztes Netz aus einer ersten Zusammenstellung von Makromeren und einer zweiten Zusammenstellung von Makromeren, wobei
die erste Zusammenstellung von Makromeren umfasst:

ein Makromer (i) mit der folgenden Strukturformel:

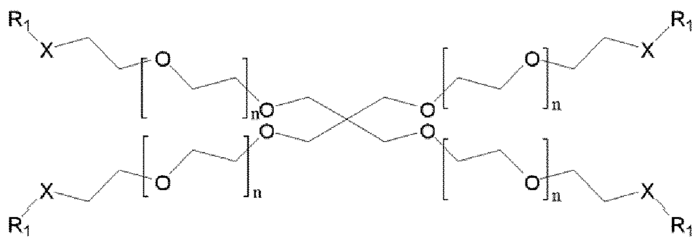

wobei
$R_1$ eine Gruppe ist, die $-N_3$ umfasst,
X eine Estergruppe ist, und
jedes n unabhängig eine ganze Zahl von 1 bis ungefähr 400 ist; und
ein Makromer (ii) mit der folgenden Strukturformel:

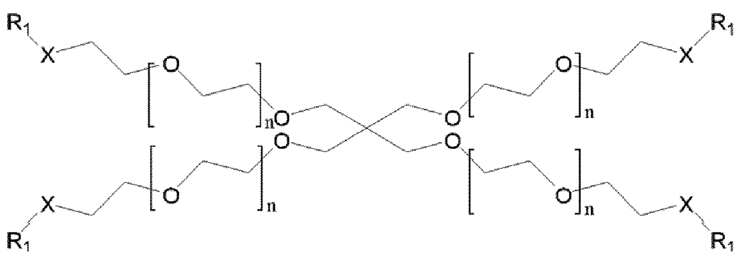

wobei
$R_1$ eine Gruppe ist, die $-N_3$ umfasst,
X leer ist, und
jedes n unabhängig eine ganze Zahl von 1 bis ungefähr 400 ist; und
die zweite Zusammenstellung von Makromeren die folgende Strukturformel aufweist:

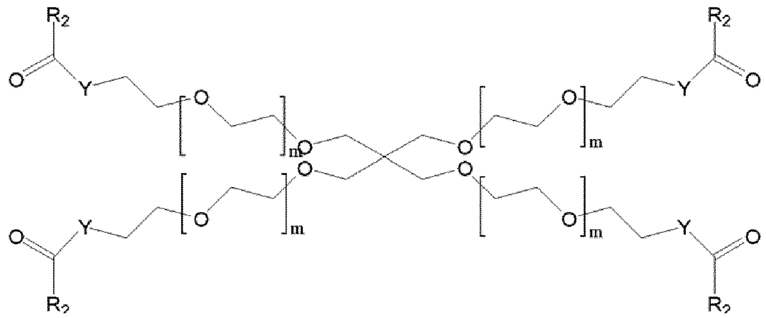

wobei
$R_2$ eine Gruppe ist, die eine cyclische oder acyclische Alkyngruppe umfasst,
Y NH, O oder leer ist, und
jedes m unabhängig eine ganze Zahl von 1 bis ungefähr 400 ist,
wobei
die erste und die zweite reaktive Endgruppe über Click-Chemie zusammengefügt sind, um ein vernetztes Netz mit einem steuerbaren und vorhersagbaren Profil der Kinetik der Gelierung oder der Zersetzung zu bilden, und die eine oder mehreren labilen und/oder stabilen Verknüpfungen in dem vernetzten Netz derart konfiguriert sind, dass sie ein steuerbares und vorhersagbares Profil der Kinetik der Gelierung oder der Zersetzung des Hydrogels bereitstellen.

**2.** Hydrogel von Anspruch 1, wobei Y NH ist.

**3.** Hydrogel von Anspruch 1, wobei Y O ist.

**4.** Hydrogel von Anspruch 1, wobei $R_2$ ist:

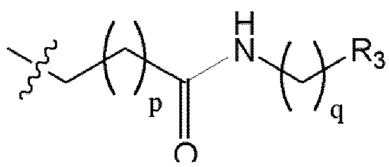

wobei $R_3$ eine Gruppe ist, die eine Gruppe umfasst, die eine cyclische oder acyclische Alkyngruppe umfasst, und jedes von p und q eine ganze Zahl von ungefähr 1 bis ungefähr 6 ist.

**5.** Hydrogel von Anspruch 4, wobei $R_3$ eine Gruppe umfasst, die ausgewählt ist aus der Gruppe bestehend aus Dibenzylcyclooctyn(DBCO)-, Dibenzocyclooctynamin-, Dibenzocyclooctyn-N-hydroxysuccinimidylester-, (1R,8S,9s)-Bicyclo[6.1.0]non-4-yn-9-ylmethanol-, (1R,8S,9s)-Bicyclo[6.1.0]non-4-yn-9-ylmethyl-N-succinimidylcarbonat- und Dibenzocyclooctynmaleimid-Gruppen.

**6.** Hydrogel von Anspruch 5, wobei das Vernetzen der ersten Zusammenstellung von Makromeren und der zweiten Zusammenstellung von Makromeren durch kupferfreie, spannungsgeförderte (strain-promoted) Azid-Alkyn-Cycloaddition oder kupferkatalysierte Azid-Alkyn-Cycloaddition durchgeführt wird.

**7.** Hydrogel-Zusammensetzung, umfassend ein Hydrogel von Anspruch 1 und ein dreidimensionales Konstrukt aus einem oder mehreren Nutzlastmaterialien, wobei das eine oder die mehreren Nutzlastmaterialien ausgewählt sind aus Proteinen, Wachstumsfaktoren, Cytokinen, rekombinanten Proteinen und Genvektoren oder einem anorganischen Material, ausgewählt aus Calciumapatiten, Calciumphosphaten. Hydroxyapatit und substituierten Hydroxyapatiten.

**8.** Vorrichtung oder Implantat, umfassend eine Hydrogel-Zusammensetzung von Anspruch 7.

**9.** Abbaubares Hydrogel mit einem steuerbaren und vorhersagbaren Profil der Kinetik der Gelierung oder der Zersetzung, umfassend ein bioorthogonal vernetztes Netz aus einer ersten Zusammenstellung von Makromeren und einer zweiten Zusammenstellung von Makromeren, wobei

die erste Zusammenstellung von Makromeren die folgende Strukturformel aufweist:

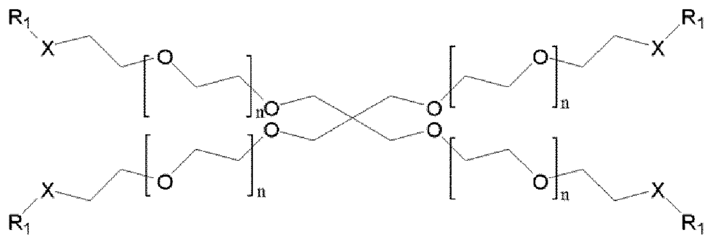

wobei
$R_1$ eine Gruppe ist, die $-N_3$ umfasst,
X eine Estergruppe oder eine Carbonatgruppe ist oder leer ist, und
jedes n unabhängig eine ganze Zahl von 1 bis ungefähr 400 ist; und
die zweite Zusammenstellung von Makromeren umfasst:

ein Makromer (iii) mit der folgenden Strukturformel:

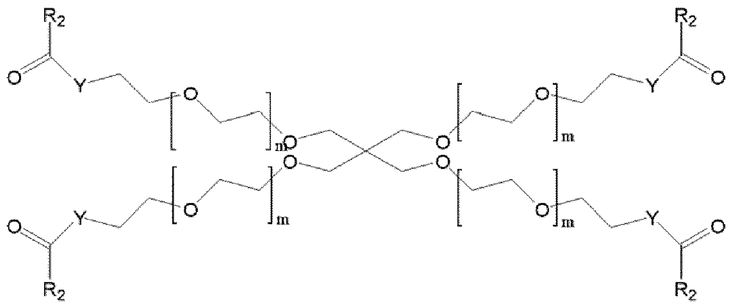

wobei
$R_2$ eine Gruppe ist, die eine cyclische oder acyclische Alkyngruppe umfasst,
Y O ist, und
jedes m unabhängig eine ganze Zahl von 1 bis ungefähr 400 ist; und
ein Makromer (iv) mit der folgenden Strukturformel:

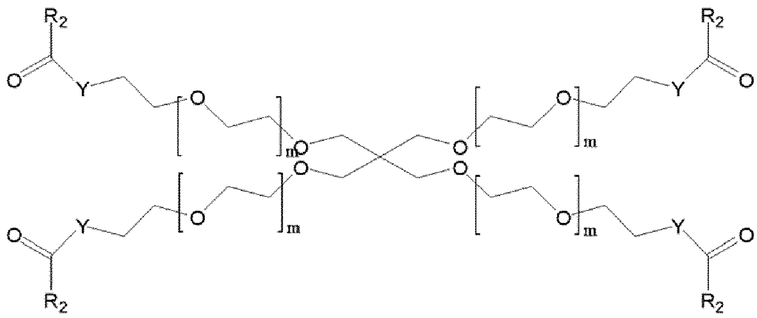

wobei
$R_2$ eine Gruppe ist, die eine cyclische oder acyclische Alkyngruppe umfasst,

Y NH oder leer ist, und
jedes m unabhängig eine ganze Zahl von 1 bis ungefähr 400 ist,
wobei die erste und die zweite reaktive Endgruppe über Click-Chemie zusammengefügt sind, um ein abbaubares vernetztes Netz mit einem steuerbaren und vorhersagbaren Profil der Kinetik der Gelierung oder der Zersetzung zu bilden,
und
die eine oder mehreren labilen und/oder stabilen Verknüpfungen in dem vernetzten Netz derart konfiguriert sind, dass sie ein steuerbares und vorhersagbares Profil der Kinetik der Gelierung oder der Zersetzung des Hydrogels bereitstellen.

10. Hydrogel von Anspruch 9, wobei $R_2$ ist:

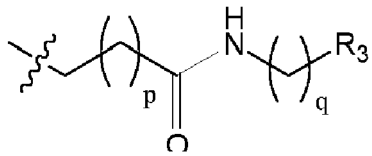

wobei $R_3$ eine Gruppe ist, die eine cyclische oder acyclische Alkyngruppe umfasst, und jedes von p und q eine ganze Zahl von ungefähr 1 bis ungefähr 6 ist.

11. Hydrogel von Anspruch 10, wobei $R_3$ eine Gruppe umfasst, die ausgewählt ist aus der Gruppe bestehend aus Dibenzylcyclooctyn(DBCO)-, Dibenzocyclooctynamin-, Dibenzocyclooctyn-N-hydroxysuccinimidylester-, (1R,8S,9s)-Bicyclo[6.1.0]non-4-yn-9-ylmethanol-, (1R,8S,9s)-Bicyclo[6.1.0]non-4-yn-9-ylmethyl-N-succinimidylcarbonat- und Dibenzocyclooctynmaleimid-Gruppen.

12. Hydrogel von Anspruch 11, wobei das Vernetzen der ersten Zusammenstellung von Makromeren und der zweiten Zusammenstellung von Makromeren durch kupferfreie, spannungsgeförderte (strain-promoted) Azid-Alkyn-Cycloaddition oder kupferkatalysierte Azid-Alkyn-Cycloaddition durchgeführt wird.

13. Hydrogel von Anspruch 9, wobei X eine Carbonatgruppe ist.

14. Hydrogel-Zusammensetzung, umfassend ein Hydrogel von Anspruch 9 und ein dreidimensionales Konstrukt aus einem oder mehreren Nutzlastmaterialien, wobei das eine oder die mehreren Nutzlastmaterialien ausgewählt sind aus Proteinen, Wachstumsfaktoren, Cytokinen, rekombinanten Proteinen und Genvektoren oder einem anorganischen Material, ausgewählt aus Calciumapatiten, Calciumphosphaten. Hydroxyapatit und substituierten Hydroxyapatiten.

15. Vorrichtung oder Implantat, umfassend eine Hydrogel-Zusammensetzung von Anspruch 14.

## Revendications

1. Hydrogel dégradable ayant une cinétique de gélification ou un profil de désintégration prévisibles et ajustables, comprenant un réseau réticulé bio-orthogonalement d’un premier ensemble de macromères et d’un second ensemble de macromères, dans lequel

le premier ensemble de macromères comprend
un macromère (i) ayant la formule développée suivante :

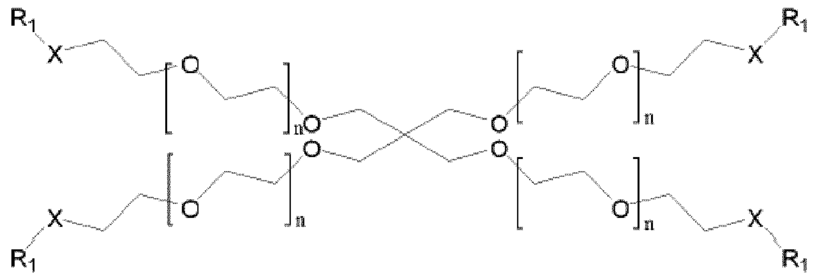

dans laquelle
$R_1$ est un groupe comprenant $-N_3$,
X est un groupe ester, et
chaque n est indépendamment un nombre entier valant de 1 à environ 400 ; et
un macromère (ii) ayant la formule développée suivante :

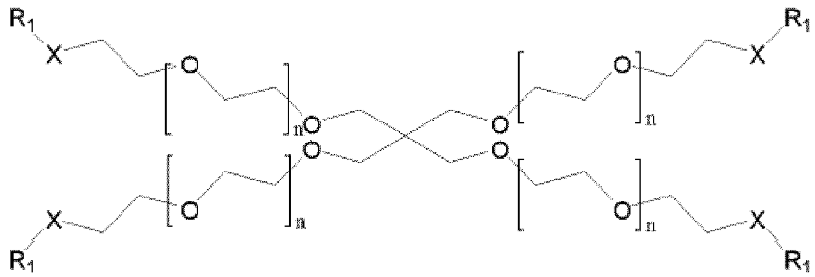

dans laquelle
$R_1$ est un groupe comprenant $-N_3$,
X est absent, et
chaque n est indépendamment un nombre entier valant de 1 à environ 400 ; et
les macromères du second ensemble de macromères ont la formule développée suivante :

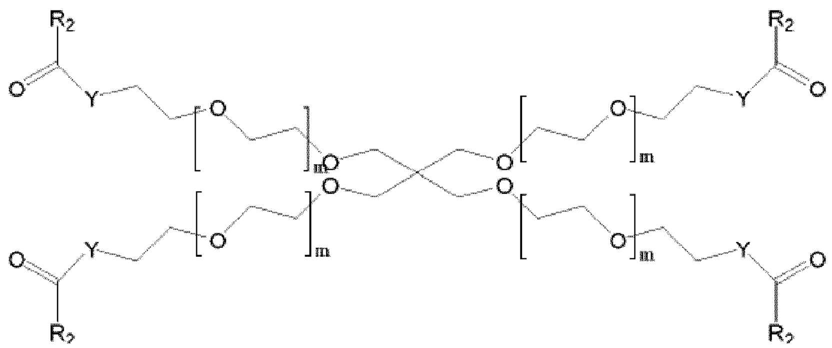

dans laquelle
$R_2$ est un groupe comprenant un groupe alcyne cyclique ou acyclique,
Y est NH, O, ou est absent, et
chaque m est indépendamment un nombre entier valant de 1 à environ 400,
dans lequel
les premier et second groupes réactifs en bout de chaîne sont joints par chimie clic pour former un réseau réticulé ayant une cinétique de gélification ou un profil de désintégration prévisibles et ajustables, et les un ou plusieurs chaînon(s) de liaison stable(s) et/ou labile(s) est/sont configuré(s) à l'intérieur du réseau réticulé de manière à fournir une cinétique de gélification ou un profil de désintégration prévisibles et ajustables de l'hydrogel.

**2.** Hydrogel selon la revendication 1, dans lequel Y est NH.

**3.** Hydrogel selon la revendication 1, dans lequel Y est O.

**4.** Hydrogel selon la revendication 1, dans lequel $R_2$ est

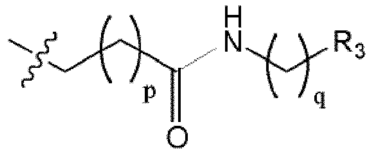

où $R_3$ est un groupe comprenant un groupe comprenant un groupe alcyne cyclique ou acyclique, p et q sont chacun un nombre entier valant d'environ 1 à environ 6.

**5.** Hydrogel selon la revendication 4, dans lequel $R_3$ comprend un groupe choisi dans le groupe constitué par les groupes dibenzylcyclo-octyne (DBCO), dibenzocyclo-octyne-amine, ester de dibenzocyclo-octyne-N-hydroxysuccinimidyle, (1R,8S,9s)-bicyclo[6.1.0]non-4-yn-9-ylméthanol, carbonate de (1R,8S,9s)bicyclo[6.1.0]non-4-yn-9-ylméthyle et de N-succinimidyle, et dibenzocyclo-octyne-maléimide.

**6.** Hydrogel selon la revendication 5, dans lequel la réticulation du premier ensemble de macromères et du second ensemble de macromères est effectuée par cyclo-addition azoture-alcyne favorisée par la tension, sans cuivre, ou cyclo-addition azoture-alcyne catalysée par du cuivre.

**7.** Composition d'hydrogel, comprenant un hydrogel selon la revendication 1 et un produit de construction tridimensionnel d'une ou de plusieurs substances de charge, lesdites une ou plusieurs substances de charge étant choisies parmi les protéines, les facteurs de croissance, les cytokines, les protéines recombinantes et les vecteurs de gènes ou une substance inorganique choisie parmi les apatites calciques, les phosphates de calcium, l'hydroxyapatite, et les hydroxyapatites substituées.

**8.** Dispositif ou implant comprenant une composition d'hydrogel selon la revendication 7.

**9.** Hydrogel dégradable ayant une cinétique de gélification ou un profil de désintégration prévisibles et ajustables, comprenant un réseau réticulé bio-orthogonalement d'un premier ensemble de macromères et d'un second ensemble de macromères, dans lequel

les macromères du premier ensemble de macromères ont la formule développée suivante :

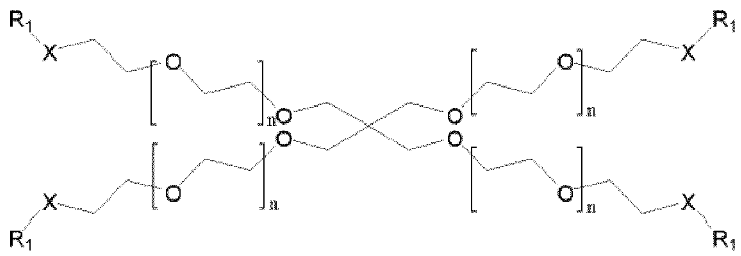

dans laquelle
$R_1$ est un groupe comprenant $-N_3$,
X est un groupe ester ou un groupe carbonate ou est absent, et
chaque n est indépendamment un nombre entier valant de 1 à environ 400 ; et
le second ensemble de macromères comprend :

un macromère (iii) ayant la formule développée suivante :

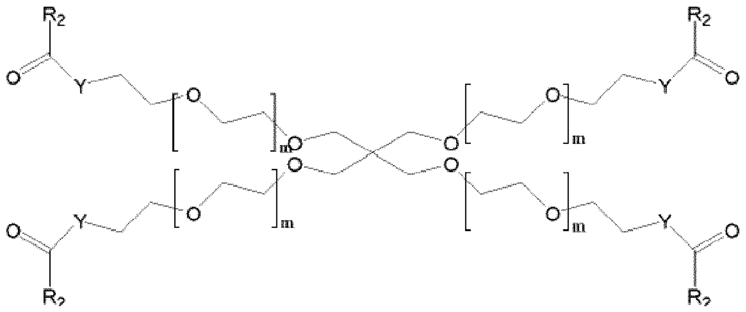

1

dans laquelle
$R_2$ est un groupe comprenant un groupe alcyne cyclique ou acyclique,
Y est O, et
chaque m est indépendamment un nombre entier valant de 1 à environ 400 ; et
un macromère (iv) ayant la formule développée suivante :

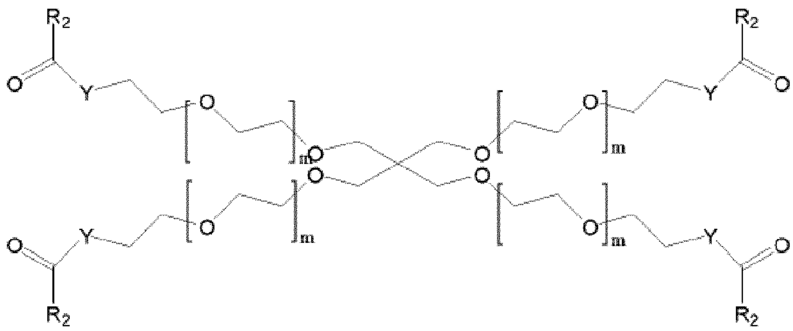

dans laquelle
$R_2$ est un groupe comprenant un groupe alcyne cyclique ou acyclique,
Y est NH ou est absent, et
chaque m est indépendamment un nombre entier valant de 1 à environ 400 ;
dans lequel les premier et second groupes réactifs en bout de chaîne sont joints par chimie clic pour former un réseau réticulé dégradable ayant une cinétique de gélification ou un profil de désintégration prévisibles et ajustables,
et
les un ou plusieurs chaînon(s) de liaison stable(s) et/ou labile(s) est/sont configuré(s) à l'intérieur du réseau réticulé de manière à fournir une cinétique de gélification ou un profil de désintégration prévisibles et ajustables de l'hydrogel.

**10.** Hydrogel selon la revendication 9, dans lequel $R_2$ est

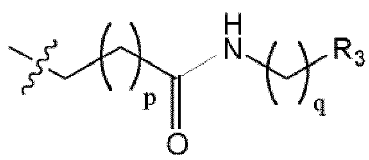

où $R_3$ est un groupe comprenant un groupe alcyne cyclique ou acyclique, p et q sont chacun un nombre entier valant d'environ 1 à environ 6.

**11.** Hydrogel selon la revendication 10, dans lequel $R_3$ comprend un groupe choisi dans le groupe constitué par les groupes dibenzylcyclo-octyne (DBCO), dibenzocyclo-octyne-amine, ester de dibenzocyclo-octyne-N-hydroxysuccinimidyle, (1R,8S,9s)-bicyclo[6.1.0]non-4-yn-9-ylméthanol, carbonate de (1R,8S,9s)-bicyclo[6.1.0]non-4-yn-9-ylméthyle et de N-succinimidyle, et dibenzocyclo-octyne-maléimide.

**12.** Hydrogel selon la revendication 11, dans lequel la réticulation du premier ensemble de macromères et du second ensemble de macromères est effectuée par cyclo-addition azoture-alcyne favorisée par la tension, sans cuivre, ou cyclo-addition azoture-alcyne catalysée par du cuivre.

**13.** Hydrogel selon la revendication 9, dans lequel X est un groupe carbonate.

**14.** Composition d'hydrogel, comprenant un hydrogel selon la revendication 9 et un produit de construction tridimensionnel d'une ou de plusieurs substances de charge, lesdites une ou plusieurs substances de charge étant choisies parmi les protéines, les facteurs de croissance, les cytokines, les protéines recombinantes et les vecteurs de gènes ou une substance inorganique choisie parmi les apatites calciques, les phosphates de calcium, l'hydroxyapatite, et les hydroxyapatites substituées.

**15.** Dispositif ou implant comprenant une composition d'hydrogel selon la revendication 14.

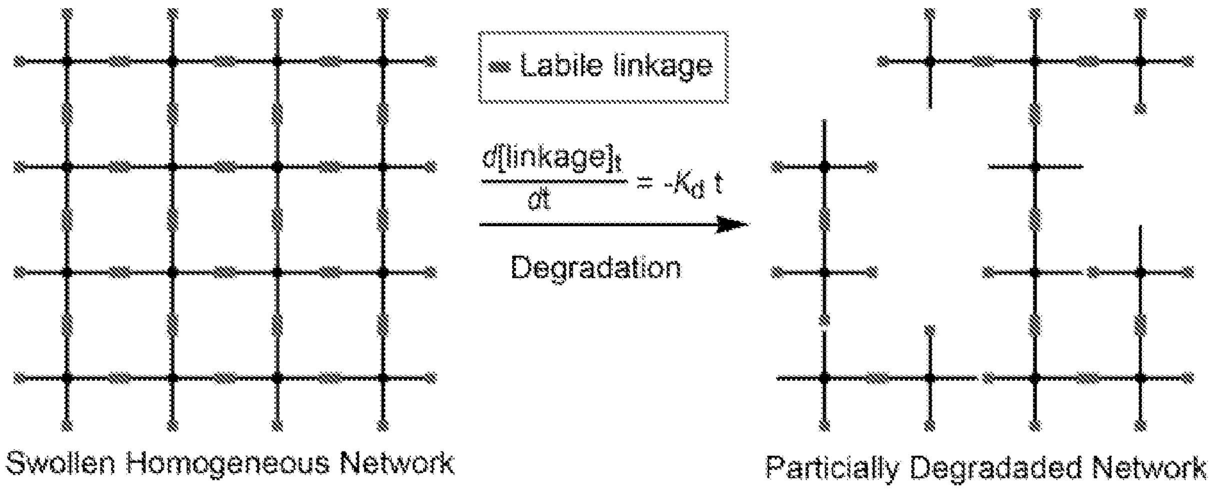


**FIG. 1**. Schematic illustration of the degradation of an ideally crosslinked and highly swollen homogeneous network containing a single labile linkage precisely positioned between each neighboring netpoints. The cleavage of the labile linkages is assumed to occur independently in a first order kinetics.

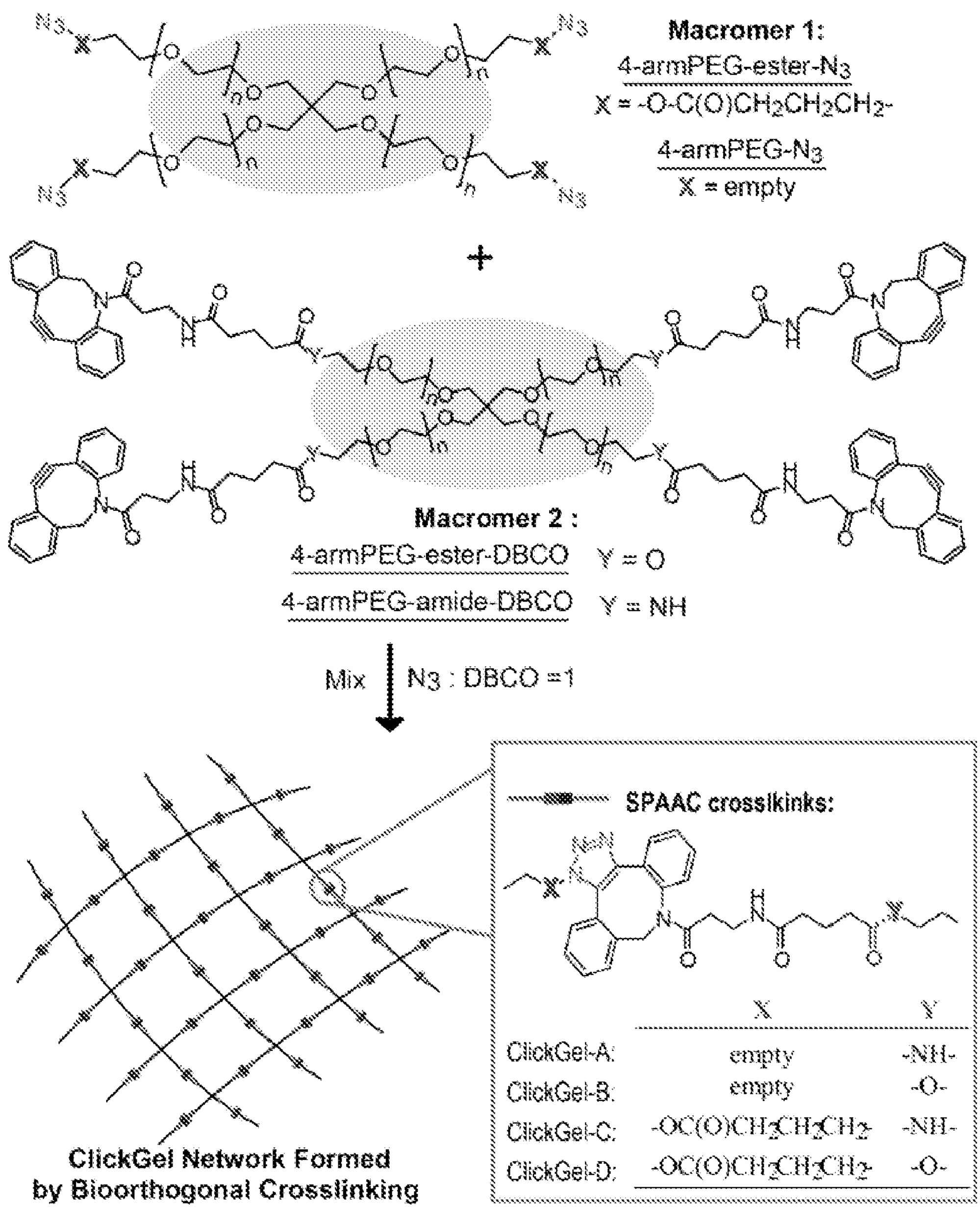


**FIG. 2.** Structures and naming of macromers and the orthogonally crosslinked ClickGel networks.

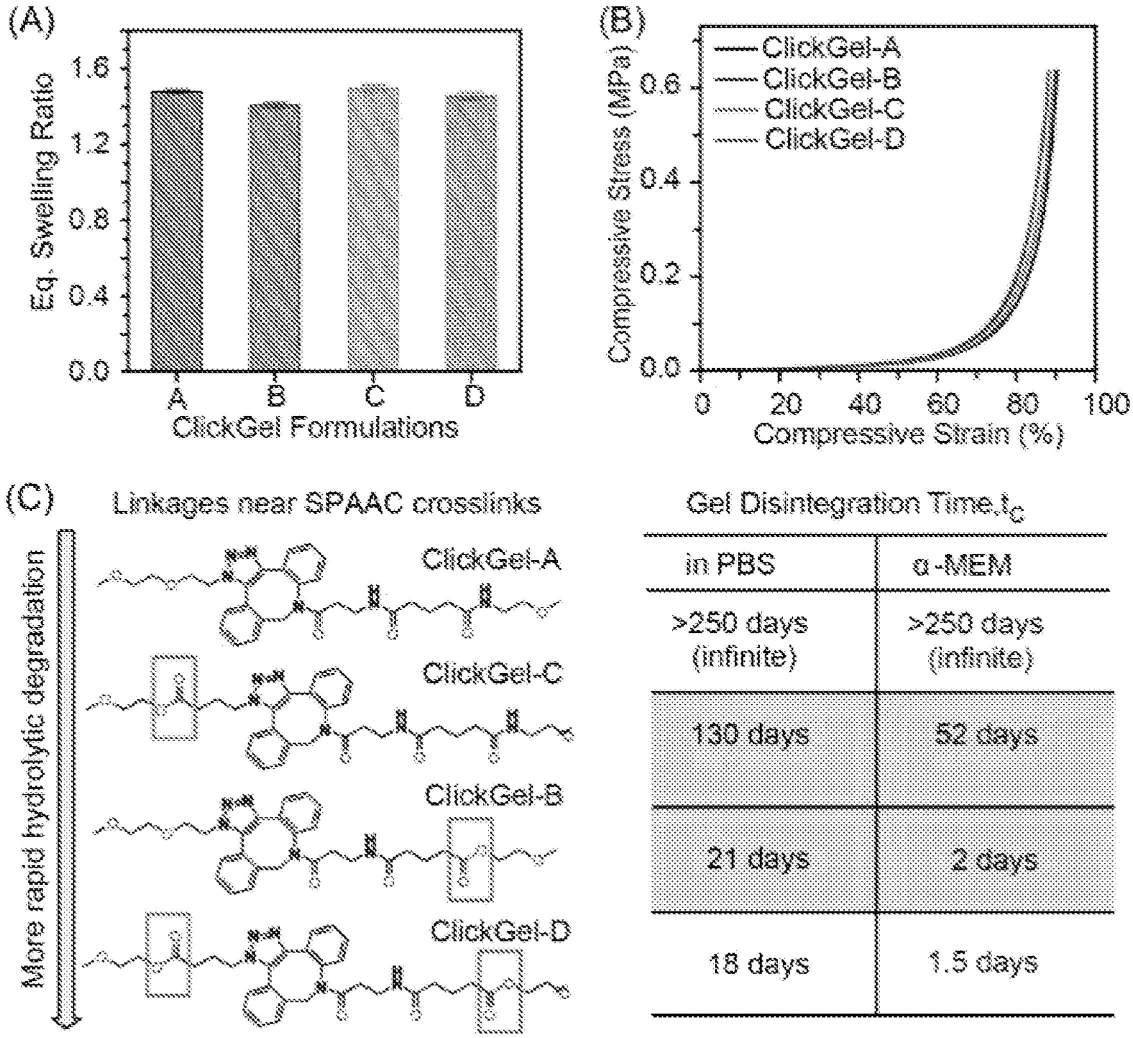


**FIG. 3.** Four hydrogels crosslinked from different combinations of azide- and DBCO-terminated macromers showing similar macroscopic properties but distinct network disintegration rates. (A) Equilibrium swelling ratio (by weight) of the four hydrogels in PBS (pH=7.4) at 37°C; (B) Stress-strain curves from unconfined compressive testing; (C) Distinct disintegration time of the hydrogels in PBS and in alpha-MEM.

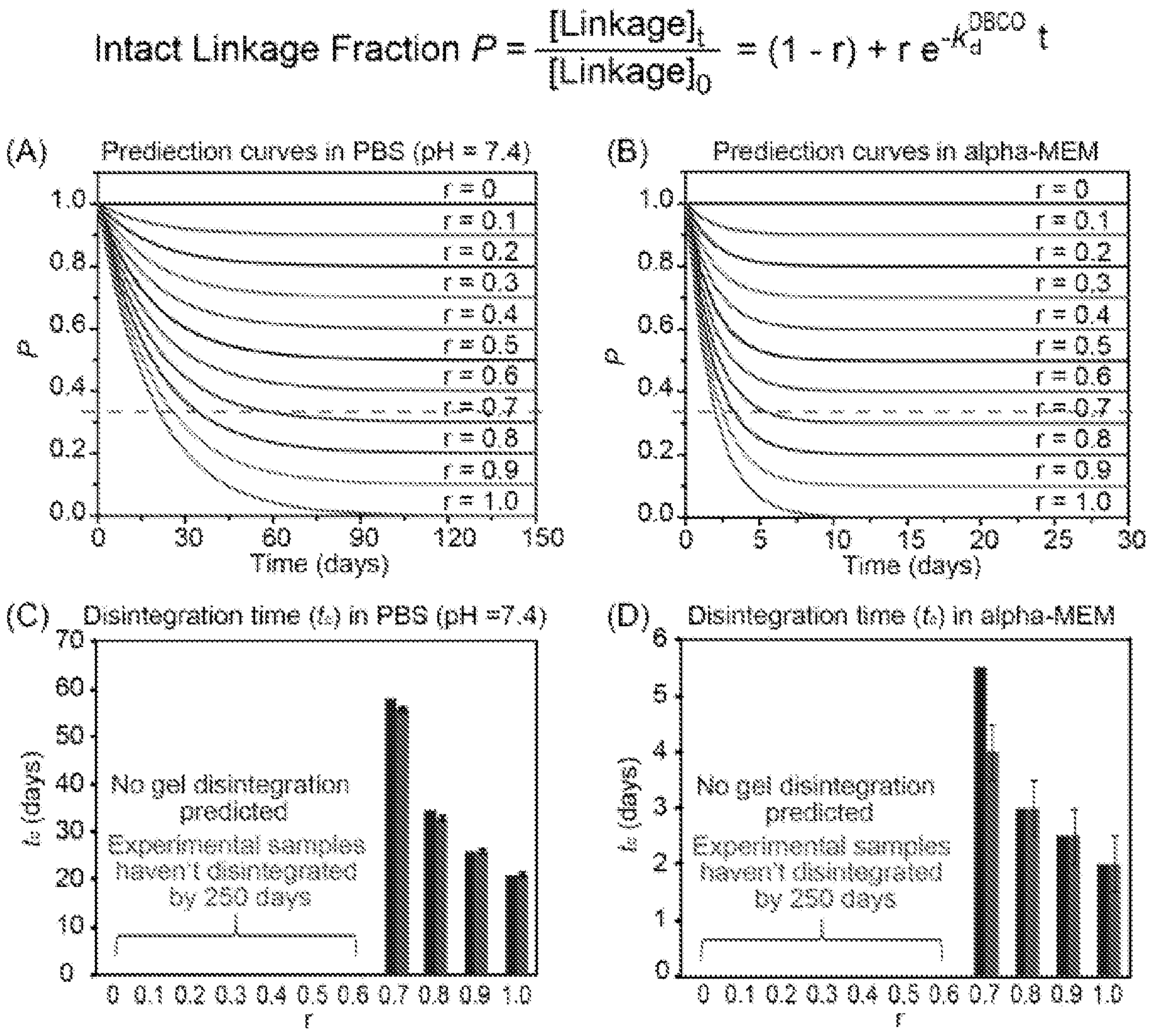


**FIG. 4.** The disintegration time ($t_c$) of a series of hydrogels prepared from 4-armPEG-$N_3$ with varying ratios of 4-armPEG-ester-DBCO and 4-armPEG-amide-DBCO (*r*) predicted by the theoretical model and validated by experimental data. (A) & (B) Theoretical prediction curves of the intact linkage fraction *P vs* time in PBS (pH=7.4) and alpha-MEM, respectively; The red dotted line represents the critical intact linkage fraction of the crosslinked 4-armPEG network ($P_c$ = 1/3), and its crosspoint with each curve indicates the predicted disintegration time for the specific formulation. (C) & (D) Predicted (blue) and experimentally observed (red) hydrogel disintegration time in PBS (pH=7.4) and alpha-MEM, respectively.

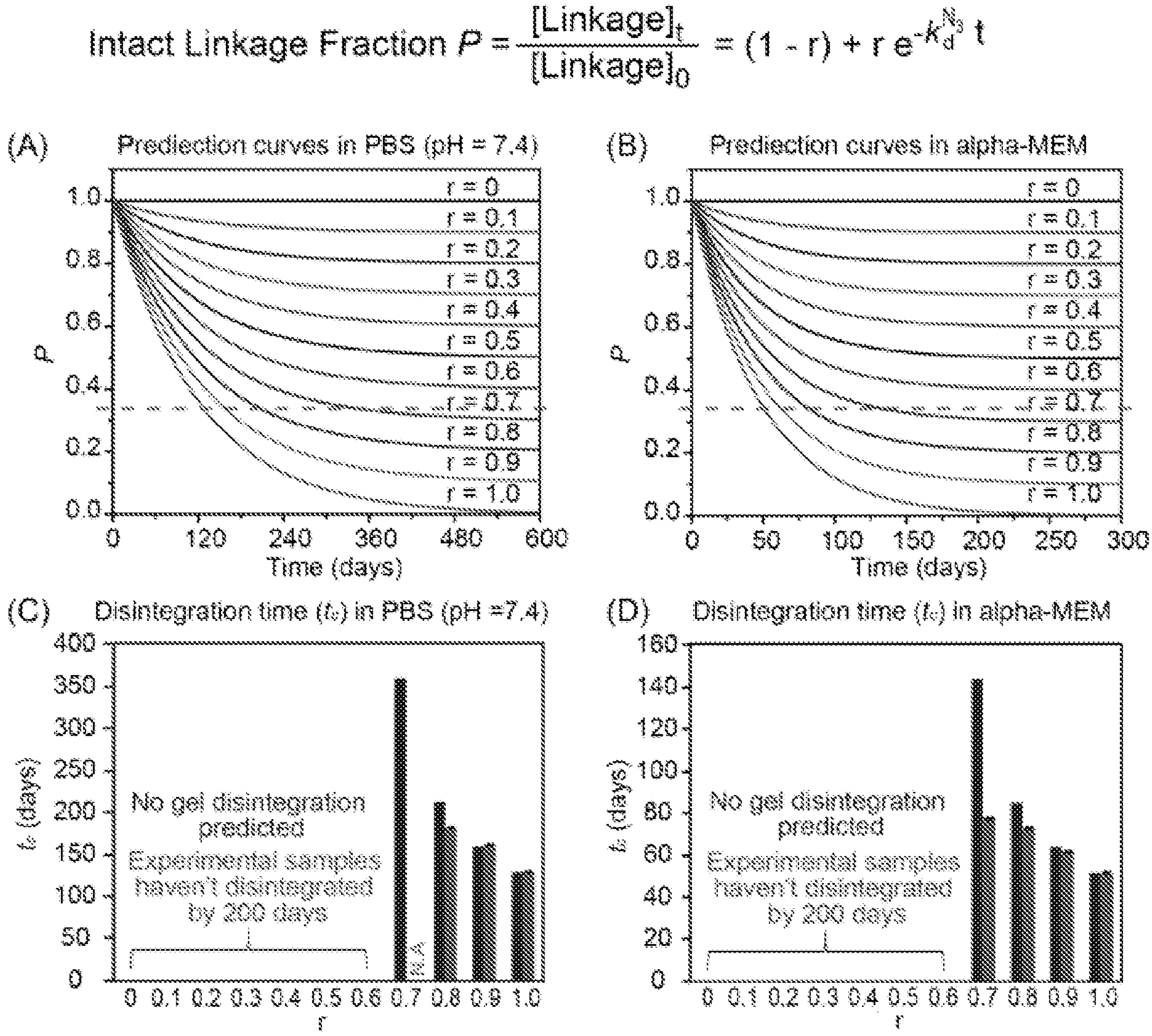


**FIG. 5.** The disintegration time ($t_c$) of a series of hydrogels prepared from 4armPEG-amide-DBCO with varying ratios of 4-armPEG-ester-$N_3$ and 4-armPEG-$N_3$ ($r$) predicted by the theoretical model and validated by experimental data. (A) & (B) Theoretical prediction curves of the intact linkage fraction $P$ *vs* time in PBS (pH=7.4) and alpha-MEM, respectively; The red dotted line represents the critical intact linkage fraction of the crosslinked 4-armPEG network ($P_c$ = 1/3), and its crosspoint with each curve indicates the predicted disintegration time for the specific formulation. (C) & (D) Predicted (blue) and experimentally observed (red) hydrogel disintegration time in PBS (pH=7.4) and alpha-MEM, respectively.

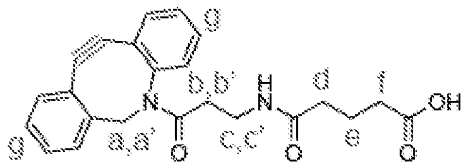

DBCO-acid

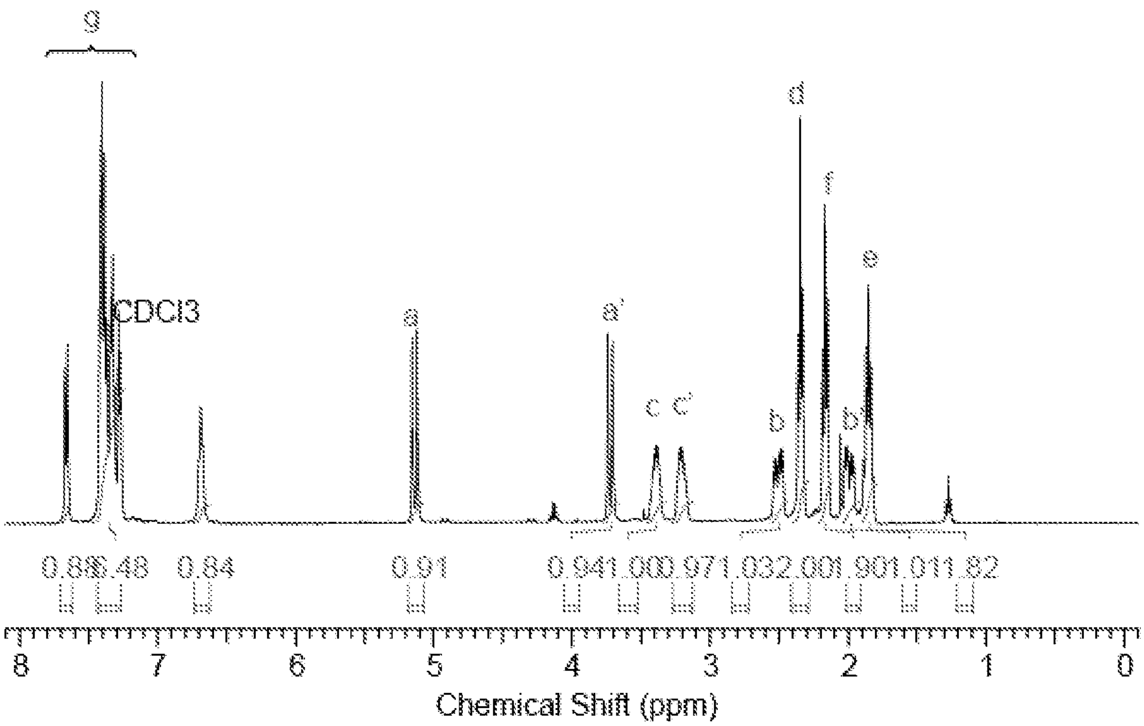


**FIG. 6**. $^1$H NMR of dibenzylcyclooctyne-acid (DBCO-acid) in $CDCl_3$.

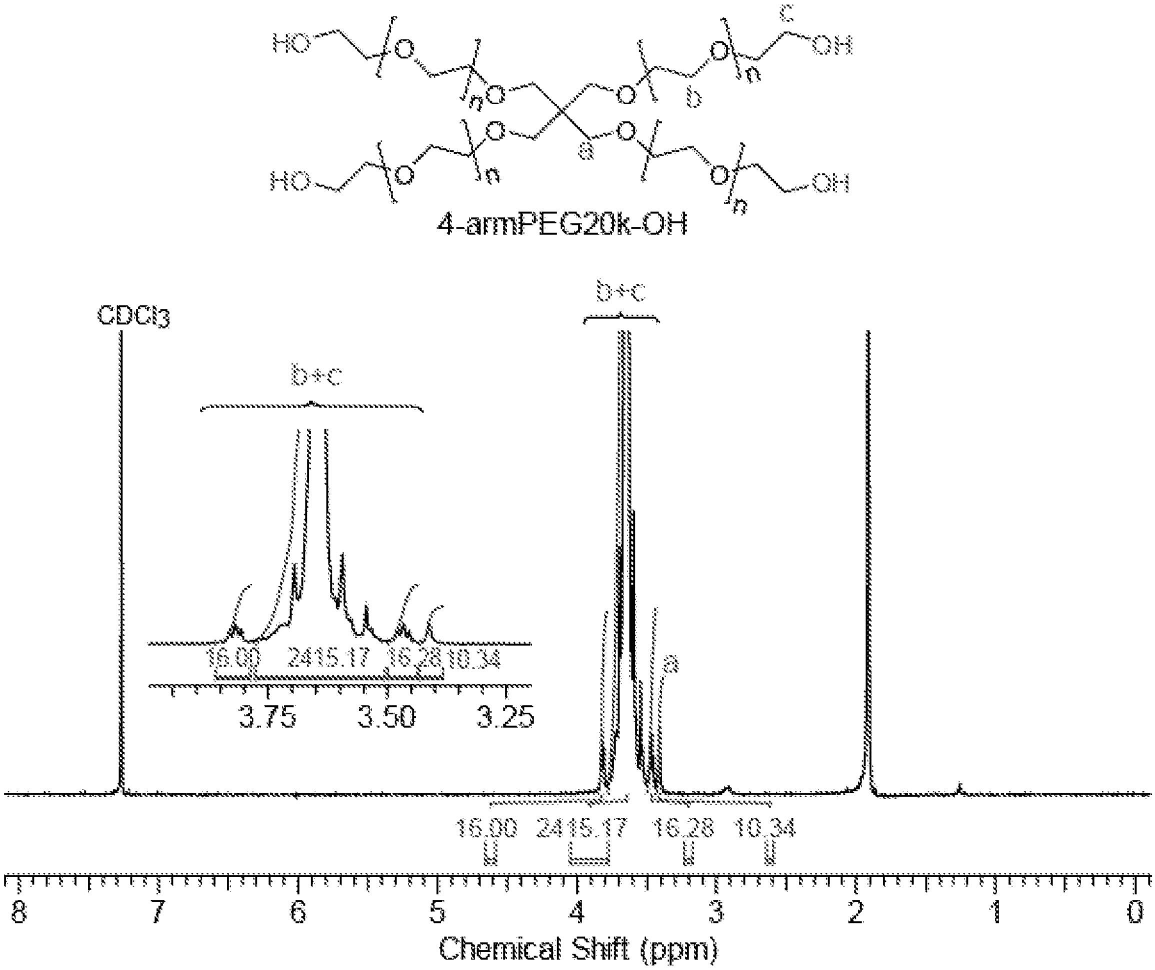


**FIG. 7**. $^{1}$H NMR of 4-armPEG20k-OH in $CDCl_3$.

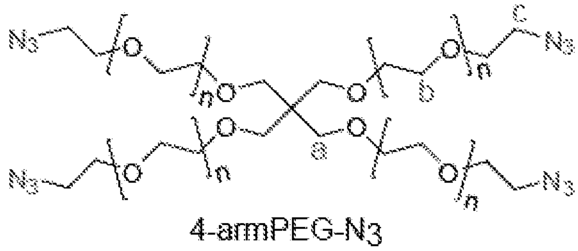

4-armPEG-$N_3$

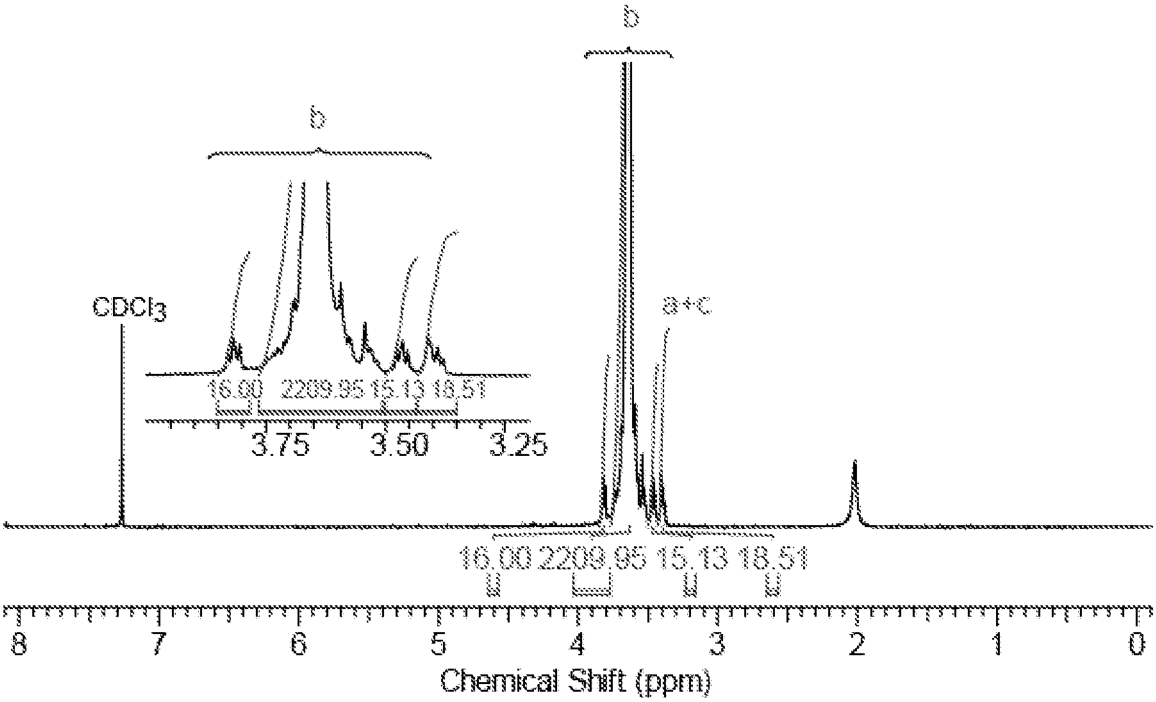


**FIG. 8**. $^1$H NMR of 4-armPEG-$N_3$ in $CDCl_3$.

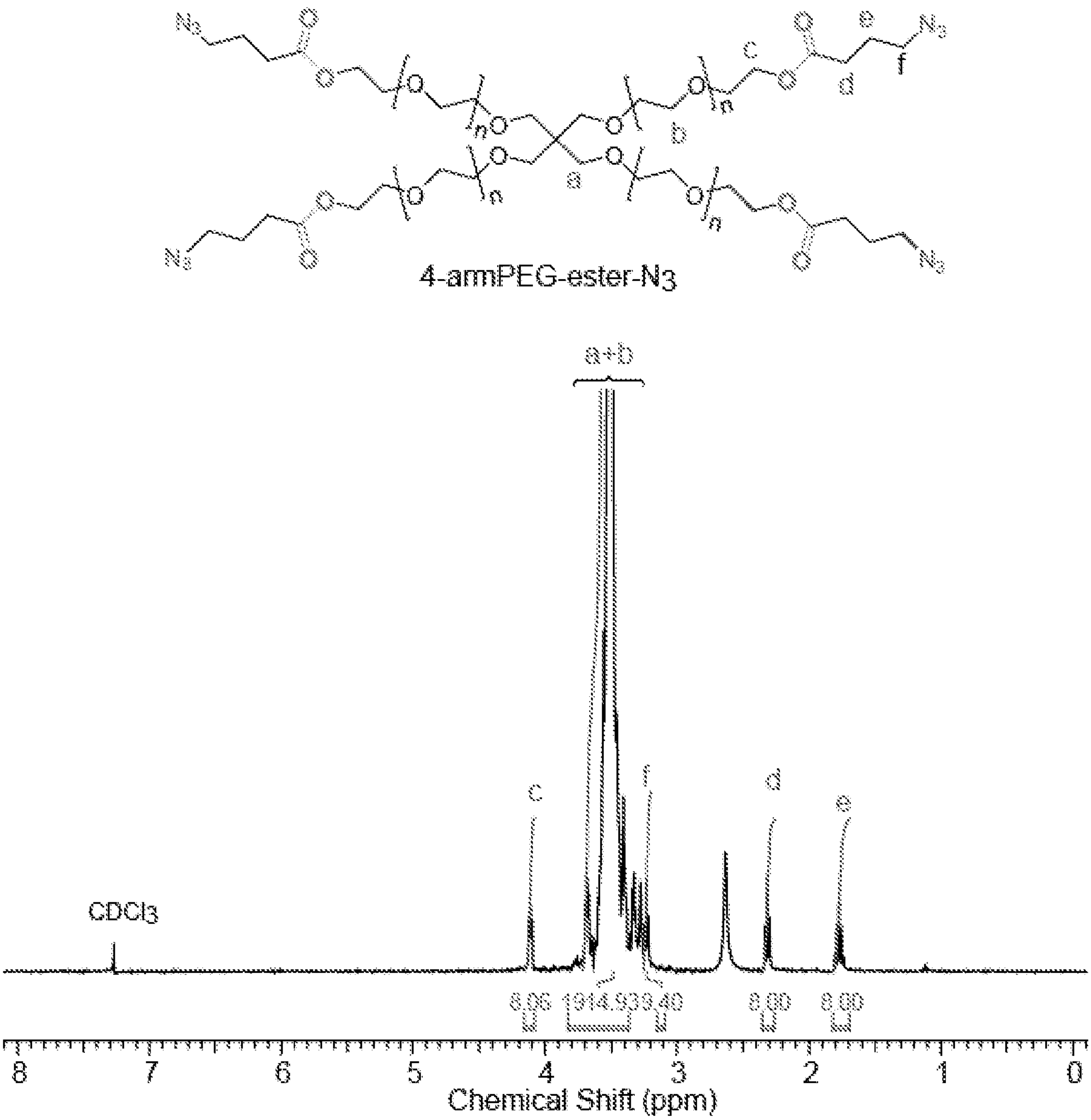
4-armPEG-ester-N3


**FIG. 9**. $^1$H NMR of 4-armPEG-ester-$N_3$ in $CDCl_3$.

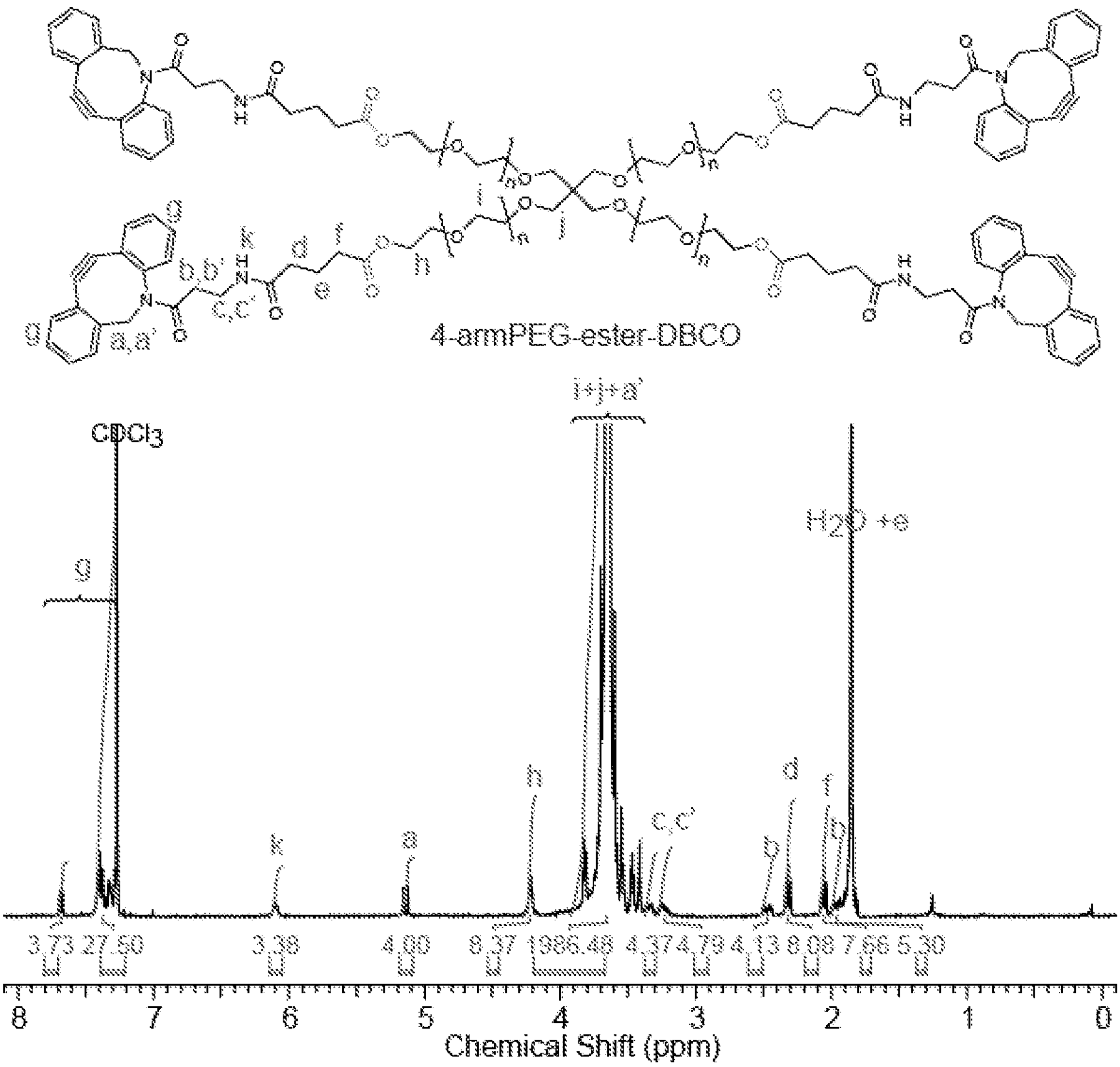
4-armPEG-ester-DBCO


**FIG. 10**. $^1$H NMR of 4-armPEG-ester-DBCO in $CDCl_3$.

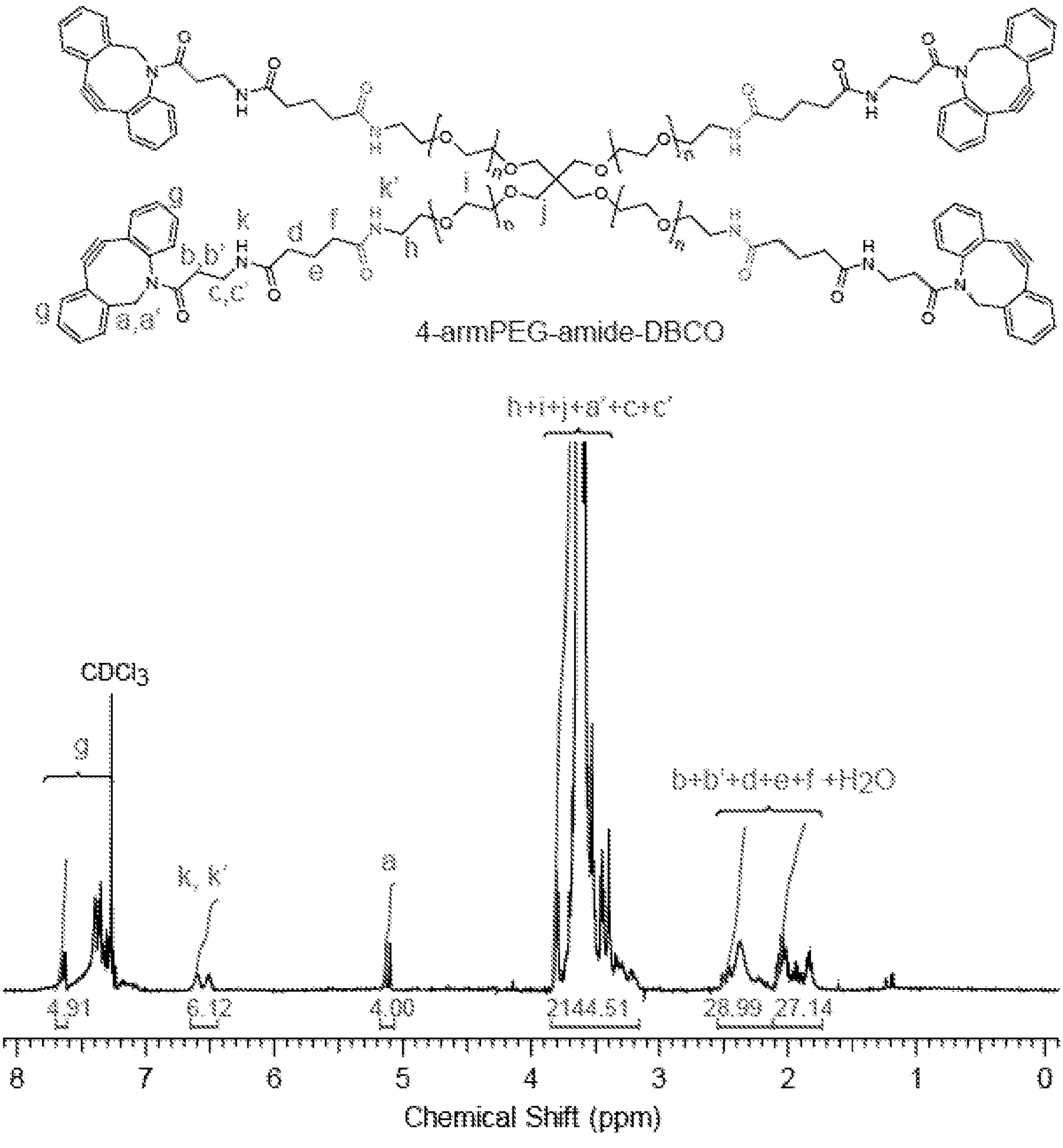


**FIG. 11**. $^1$H NMR of 4-armPEG-amide-DBCO in $CDCl_3$.

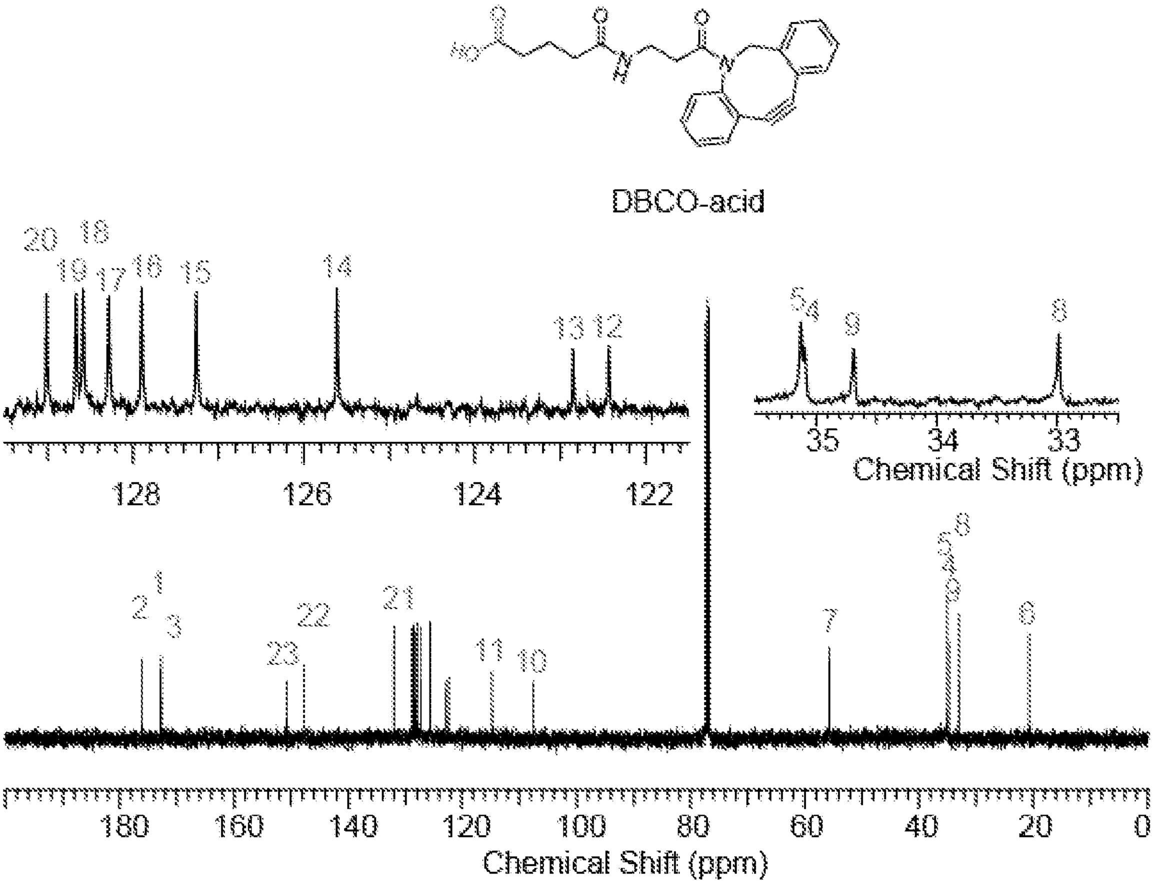


**FIG. 12**. $^{13}C$ NMR of dibenzylcyclooctyne-acid (DBCO-acid) in $CDCl_3$.

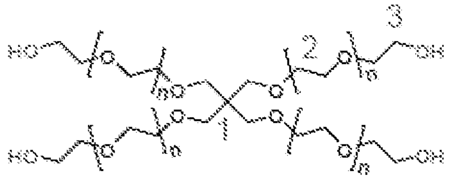

4-arm-PEG20k-OH

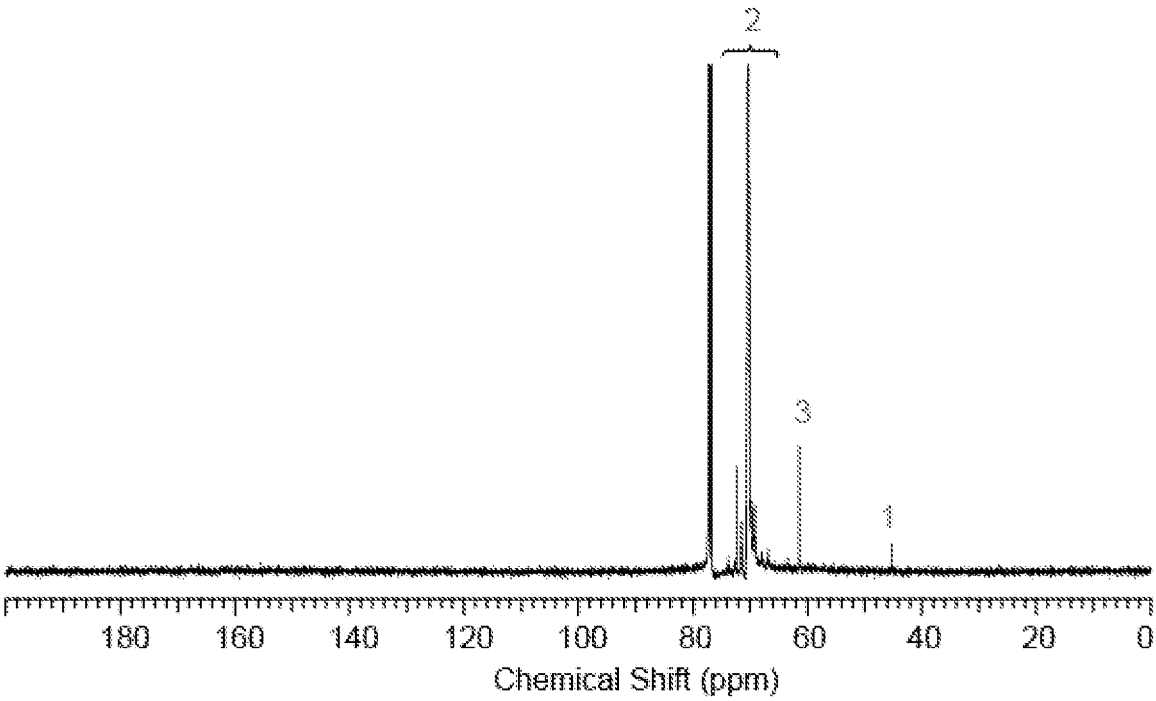


**FIG. 13**. $^{13}C$ NMR of 4-armPEG20k-OH in $CDCl_3$.

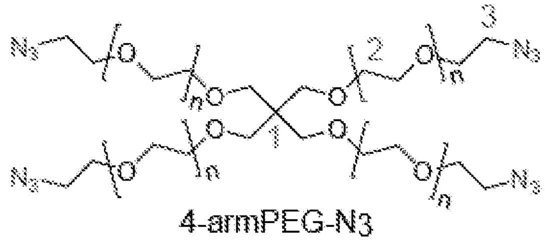

4-armPEG-N3

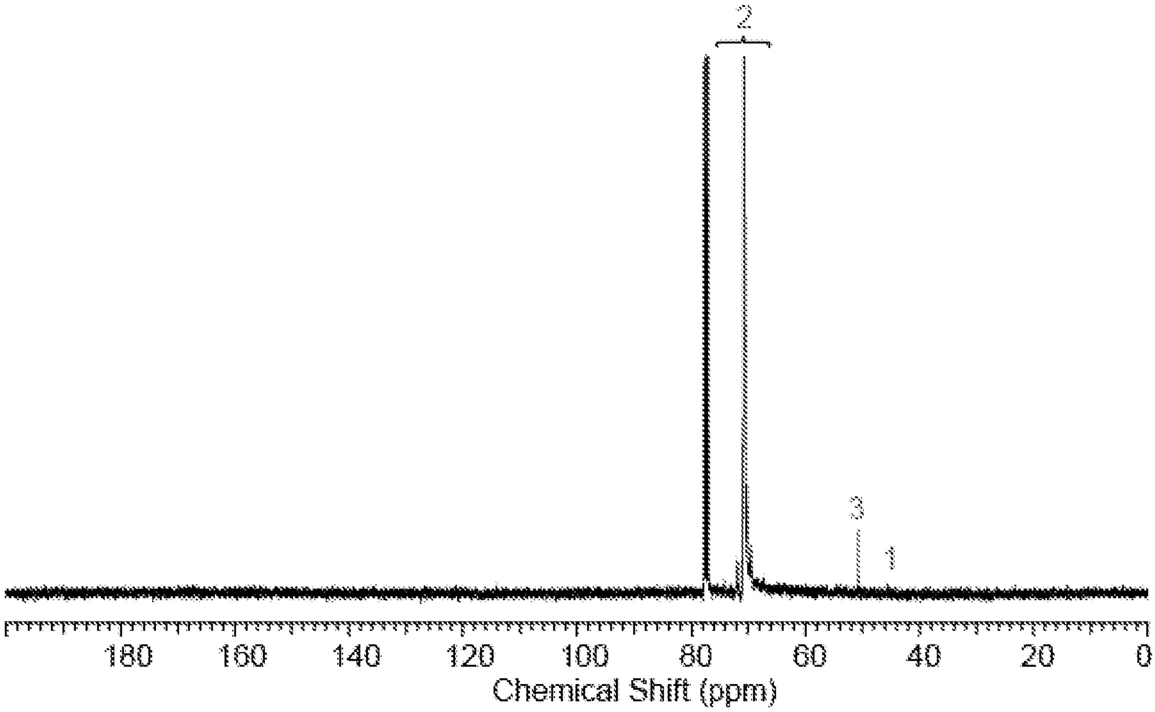


**FIG. 14**. $^{13}C$ NMR of 4-armPEG-$N_3$ in $CDCl_3$.

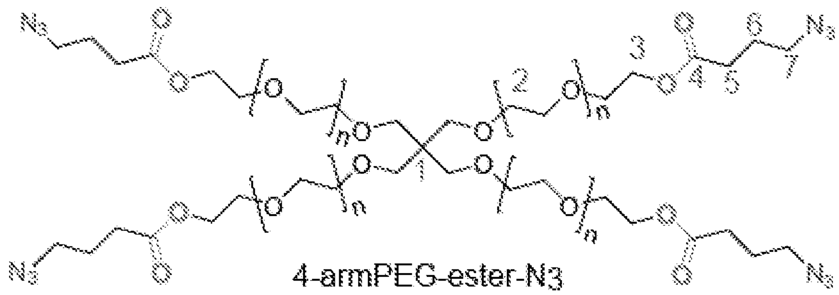

4-armPEG-ester-N3

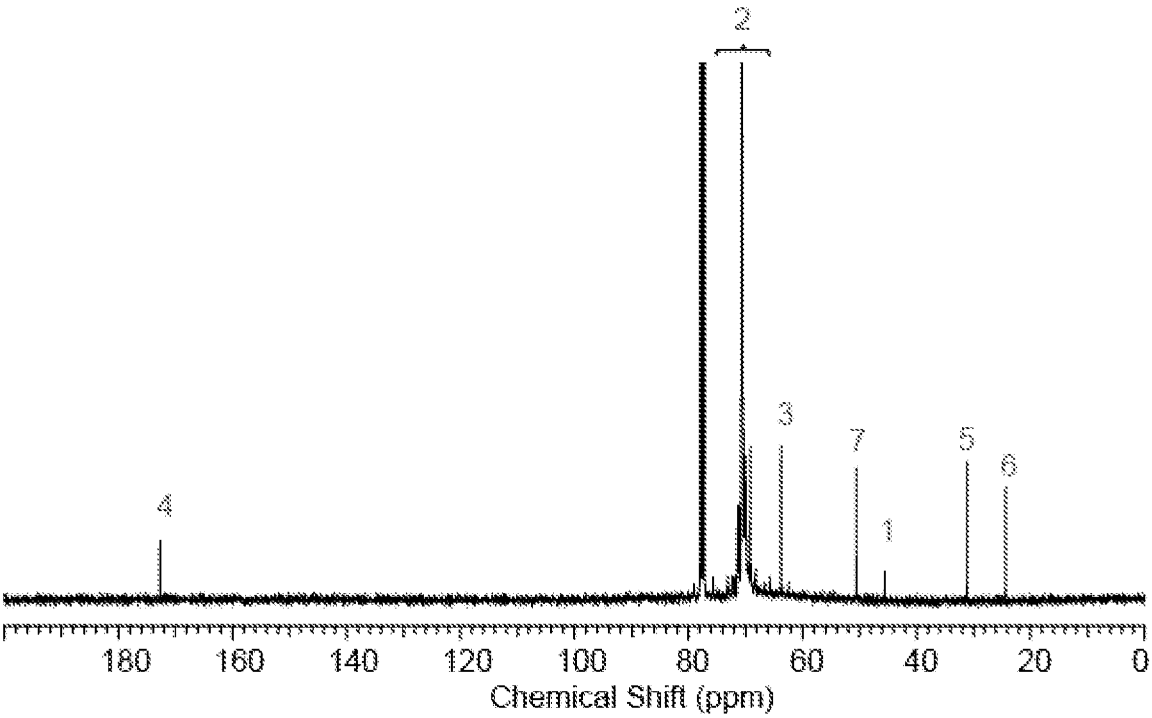


**FIG. 15**. $^{13}C$ NMR of 4-armPEG-ester-$N_3$ in $CDCl_3$.

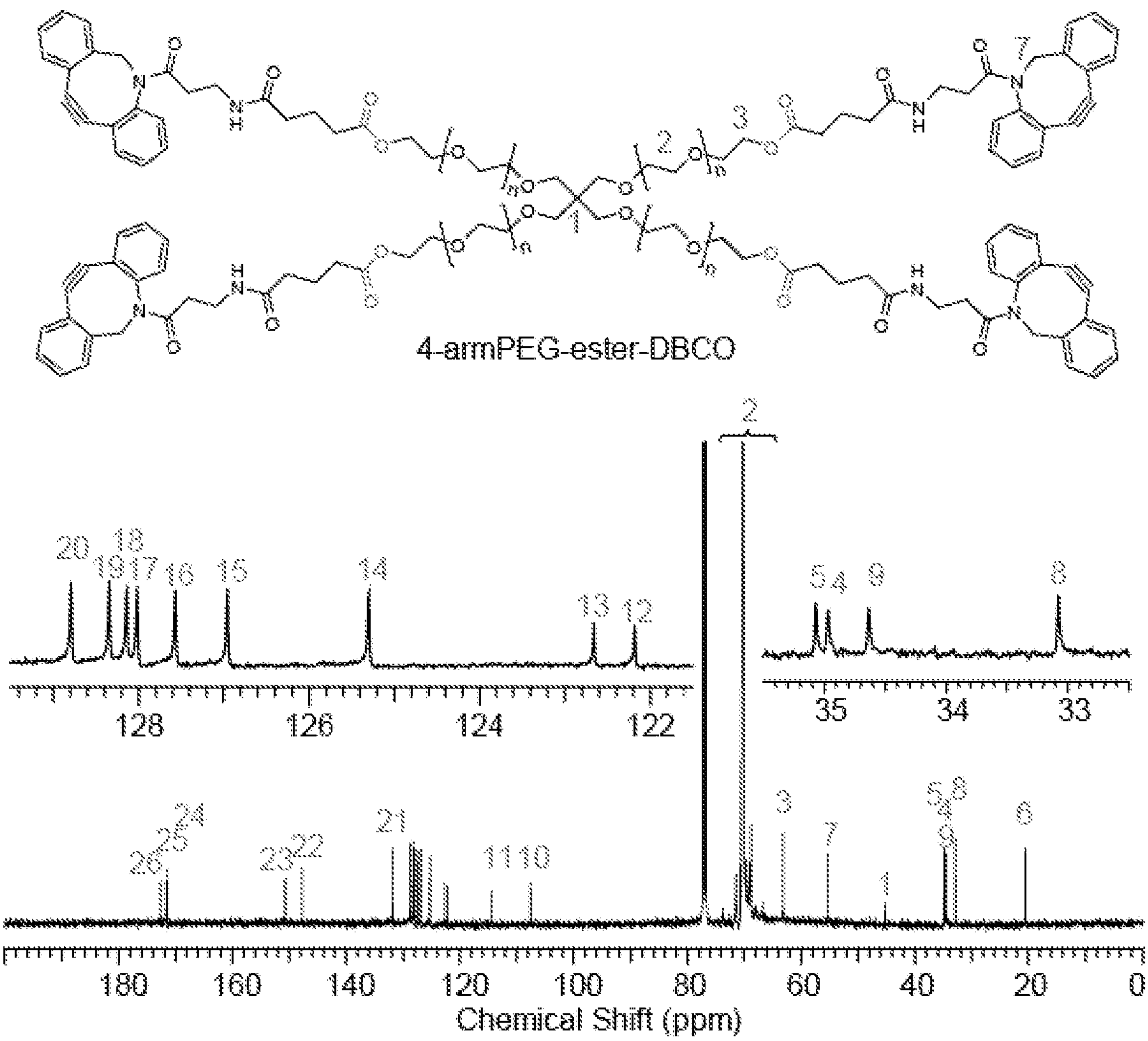
4-armPEG-ester-DBCO


**FIG. 16**. $^{13}C$ NMR of 4-armPEG-ester-DBCO in $CDCl_3$.

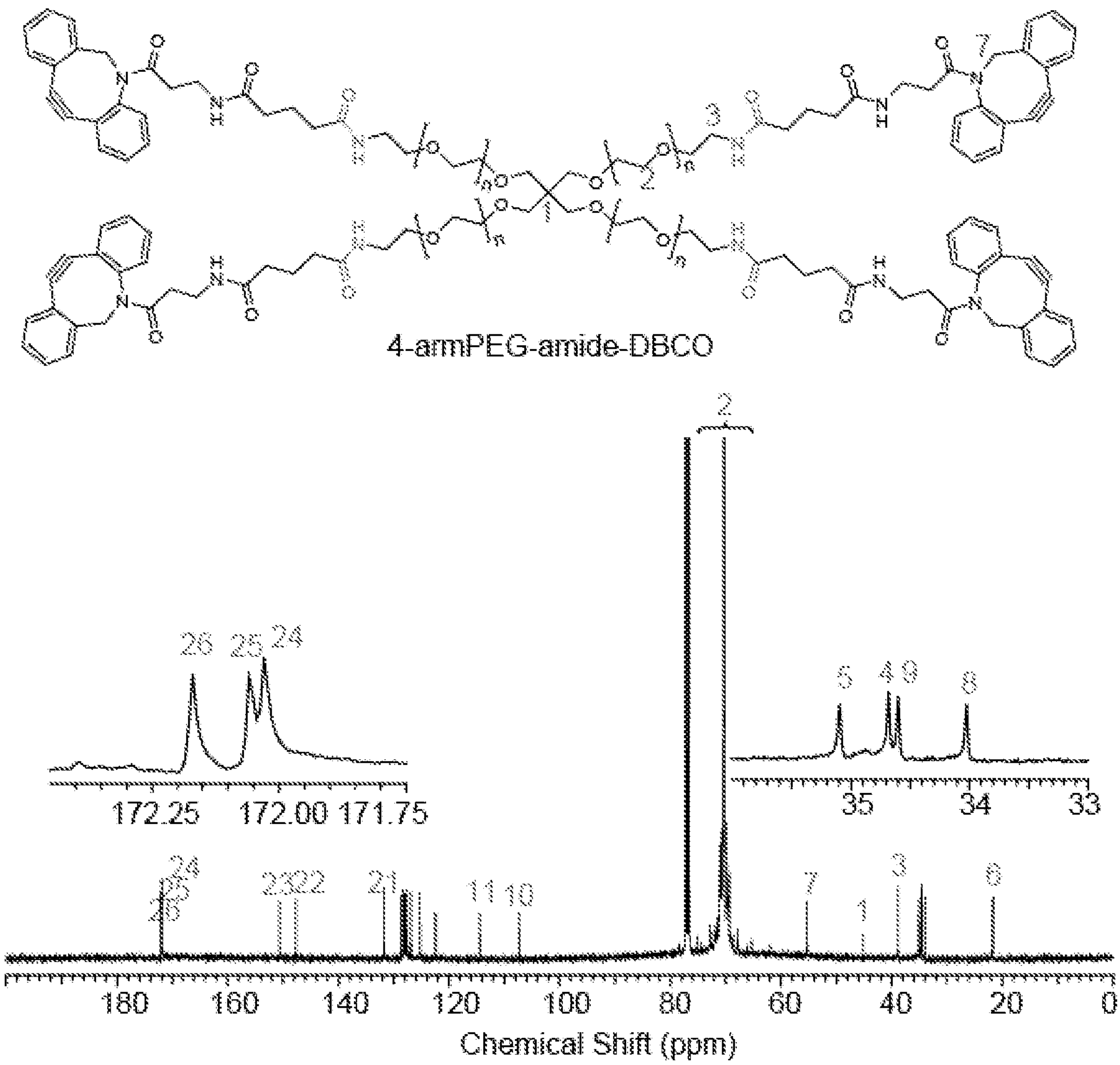


**FIG. 17**. $^{13}C$ NMR of 4-armPEG-amide-DBCO in $CDCl_3$.

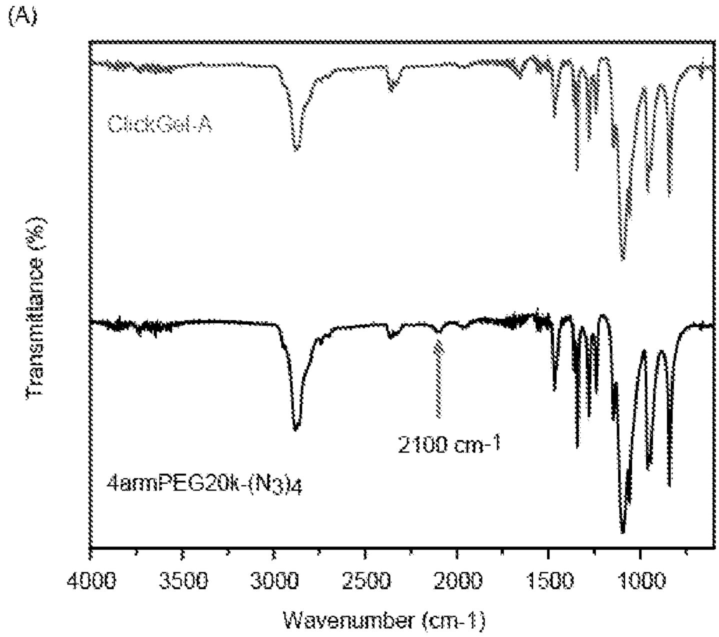


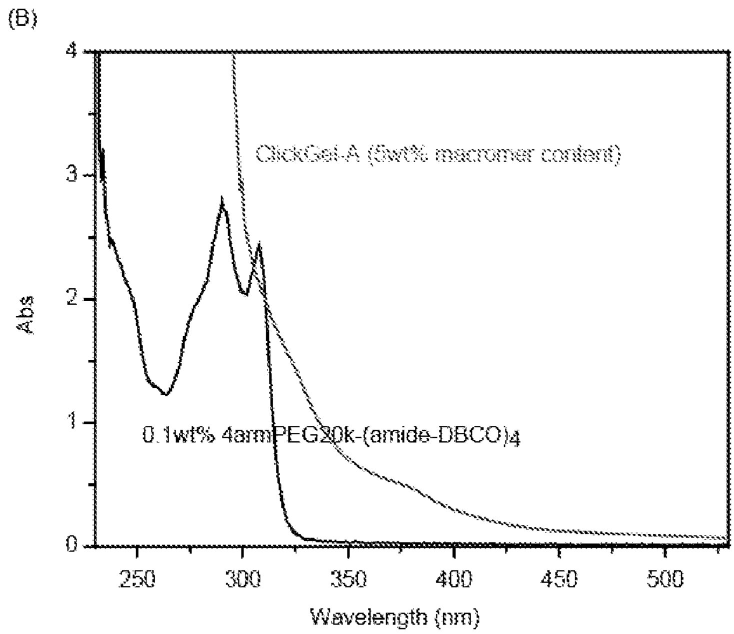


**FIG. 18**. Nearly complete conversion of reactive functional groups confirmed by spectroscopic measurements. (a) FTIR showing the characteristic peak of azide group at 2100cm$^{-1}$ in azido-containing macromer completely disappeared upon gelling of the hydrogel; and (b) UV-vis measurement showing the characteristic absorption at 307 nm for alkyne group in ClickgGel-A with 5 wt% macromer content is lower than that of the 0.1wt 4-armPEG-amide-DBCO macromer solution.

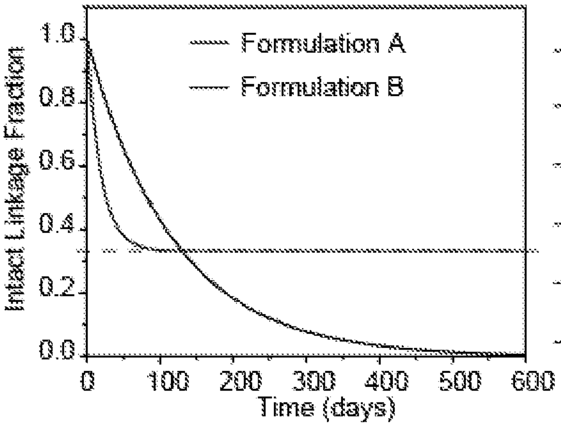


| Formulation | A | B |
|---|---|---|
| 4-armPEG-ester-$N_3$ | 1 | 0 |
| 4-armPEG-$N_3$ | 0 | 1 |
| 4-arm-PEG-ester-DBCO | 0 | 0.667 |
| 4arm-PEG-amide-DBCO | 1 | 0.333 |

**FIG. 19.** A demonstration that hydrogels with the same disintegration time but different degradation profiles could be obtained through different formulations enabled by the versatile hydrogel platform. The blue line is the predicted degradation curve for Formulation A, while the black line is the predicted degradation curve for Formulation B. The red dotted line in the plot represents the critical intact linkage fraction ($P_c$) of 1/3 to reach network disintegration.

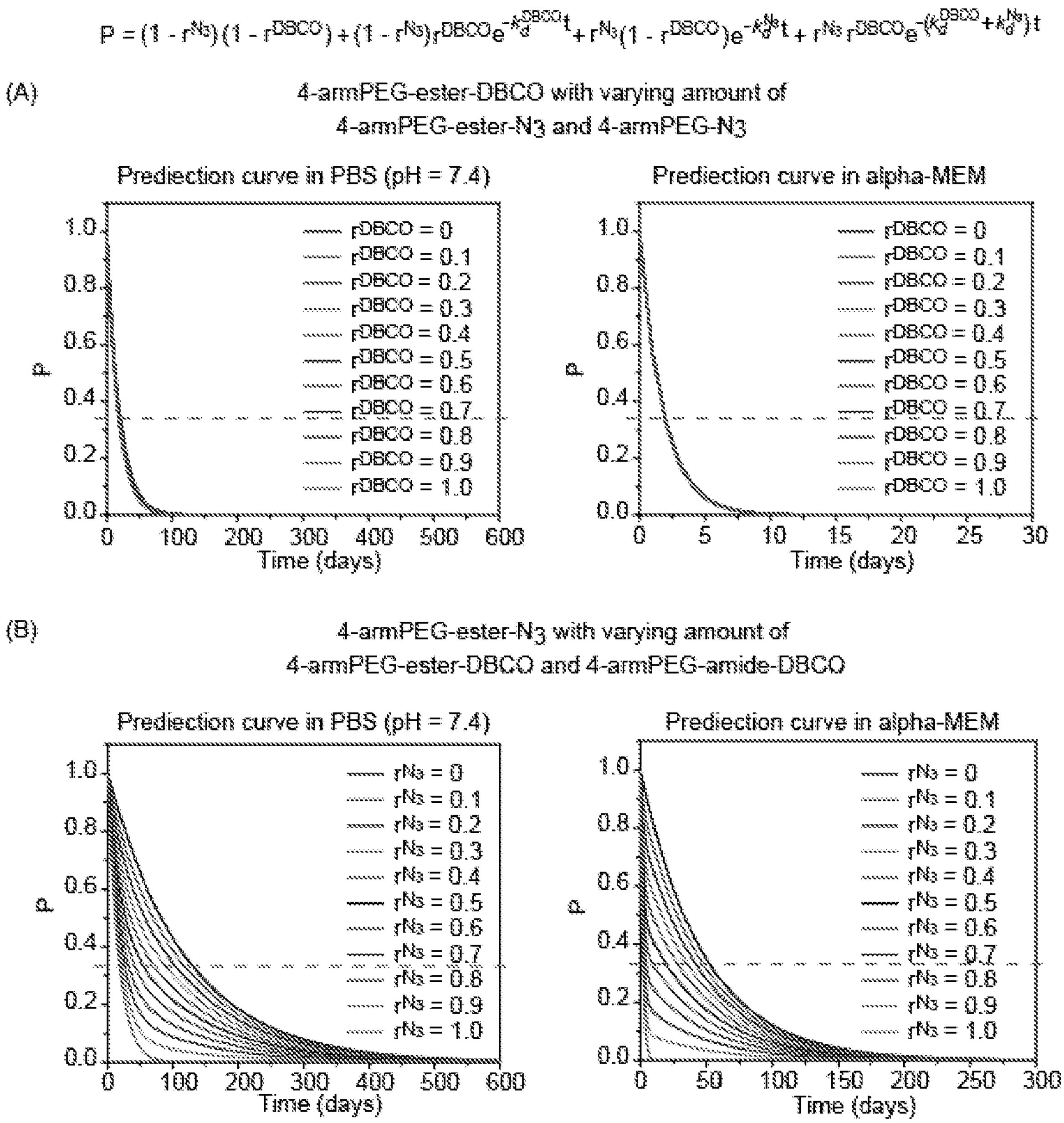


**FIG. 20.** Examples of a wide range of degradation profiles obtainable with the versatile hydrogel platform based on the simple model involving two formulation-dependent parameters. (A) The prediction curves of intact labile linkage fraction (*P*) over time in PBS and in aMEM for formulations with $r^{N3} = 0$ but varying $r^{DBCO}$; (B) The prediction curves of the intact linkage fraction (*P*) over time in PBS and cell culture media for formulations with $r^{DBCO} = 0$ but varying $r^{N3}$. The red dotted line in the plots represent the critical intact linkage fraction ($P_c$) of 1/3 for reaching network disintegration.

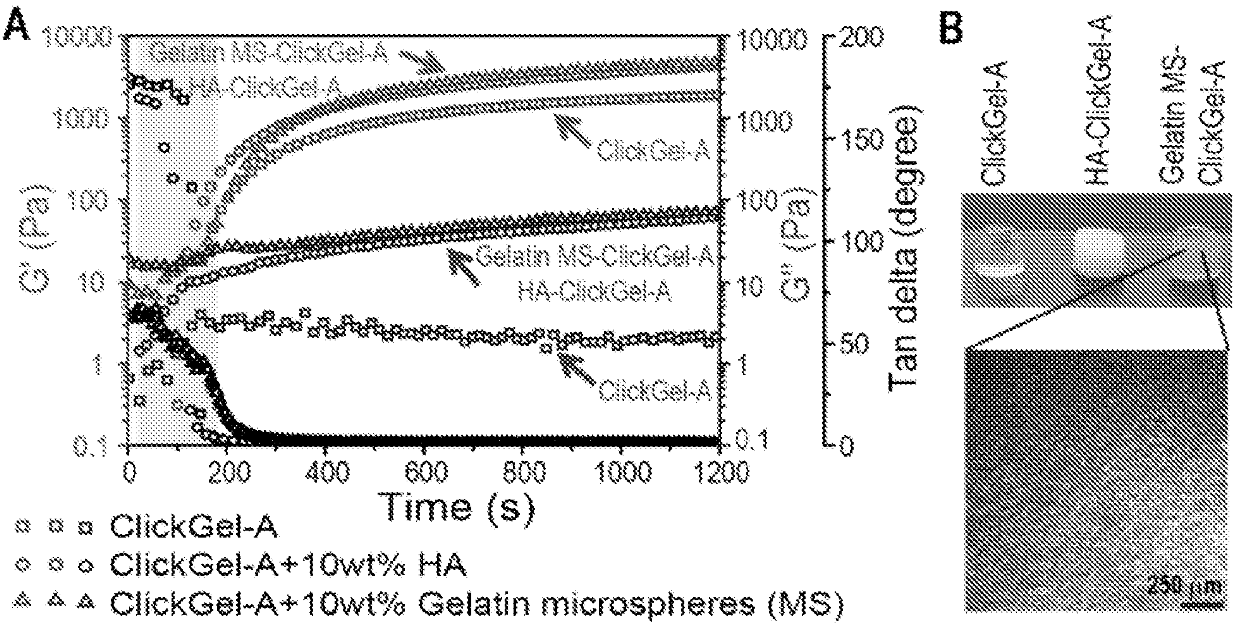


**FIG. 21.** (A) Time-dependent shear storage moduli (G', red), shear loss moduli (G'', blue) and loss tangent (tan delta, black), (B) photo/micrographs of ClickGel-A and HA-ClickGel-A or gelatin MS-ClickGel-A composites. PBS solutions of 5wt% 4armPEG20k-(amide-DBCO)$_4$ and 4armPEG20k-(N$_3$)$_4$, with/without 10wt% HA or gelatin MS were mixed at rt and loaded on bottom parallel plate of AR-2000 rheometer. Data acquisition started at 2 min (10 rad/s oscillatory angular frequency, 10% stain, 22 °C). Yellow zone indicates gelling timeframe.

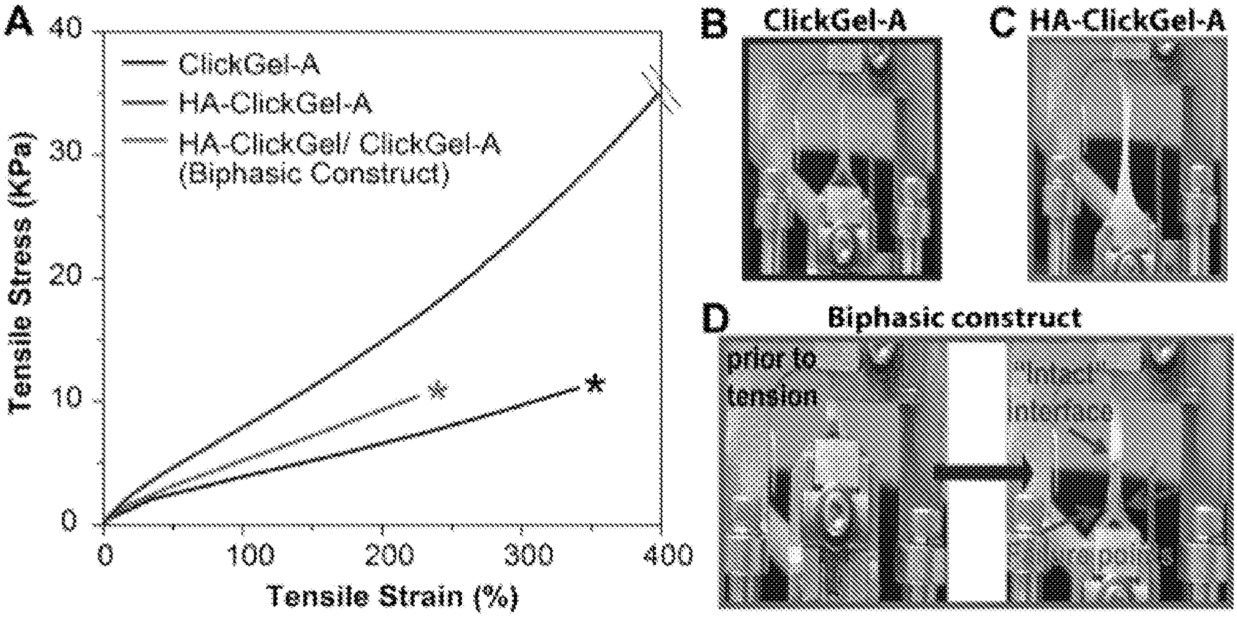


**FIG. 22.** (A): Stress-stain curve of ClickGel-A (20×3×3mm$^3$), 10wt% HA-ClickGel-A composite, and a biphasic construct composed of these compositions (bottom macromers/HA mixture was gelled for 4 min before addition of top phase macromers). All specimens were cured at rt for 4 h before tensile testing on a Q800 DMA (force ramping to 18 N at 1 N/min). *Failure point; (B)-(D): Photographs of strained specimens.

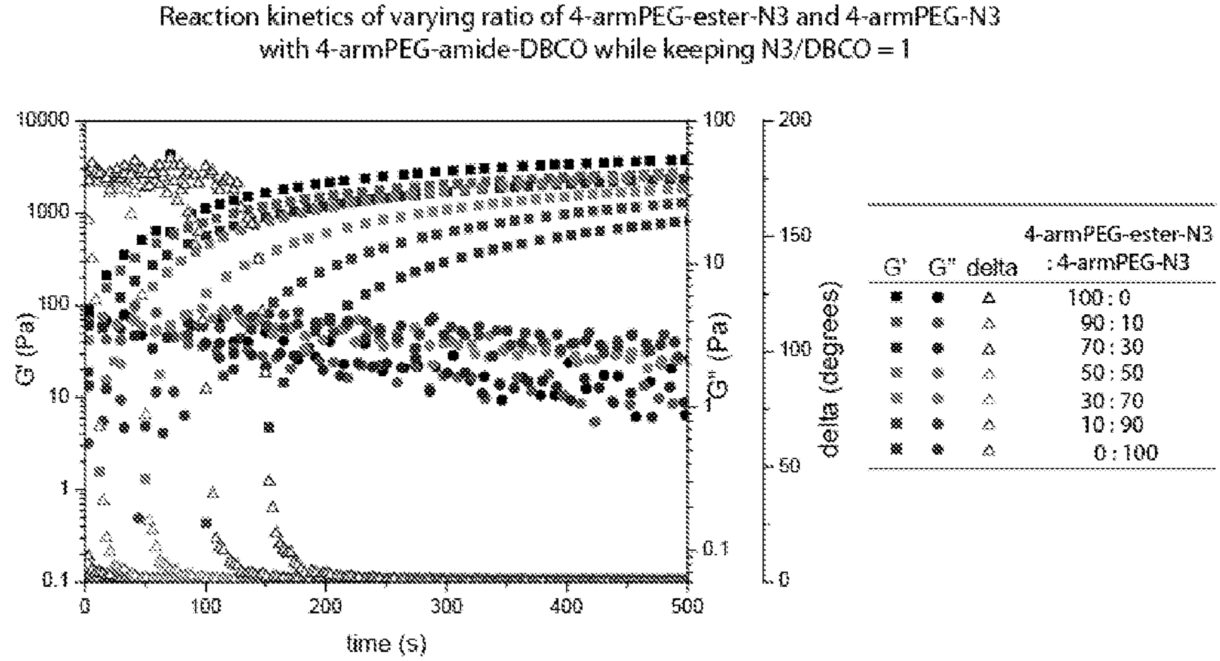


**FIG. 23.** Gelling kinetics of ClickGel can be altered by varying the ratio of azido-macromers with varying neighboring linker to the azido end group mixed with DBCO-terminated macromers. The gelling time, determined from the cross-point of G' and G" curves plus 2-min for loading mixture to the rheometer, can be tuned from less than 2 min to 5min by increasing the percentage of 4-armPEG-$N_3$ in the total $N_3$-terminated macromers. The dynamic rheology test was performed on an AR-2000 rheometer (TA Instruments) equipped with 8-mm parallel plates and a Peltier heating unit. The gelling process of the various formulations and the evolution of the shear modulus of the hydrogels were studied by oscillatory time sweep rheology experiments at 37 °C. Aqueous solutions of azido-terminated and DBCO-terminated 4-arm PEG macromers (5 w/w%) in PBS (pH =7.4) with 1:1 molar ratio of the azide groups to the DBCO groups were loaded on the bottom plate sequentially and mixed by pipette. The experiment and data collection were initiated 2 minutes after mixing to ensure consistency among various formulations. An oscillatory angular frequency of 10 rad/s and strain of 10% were applied.

A

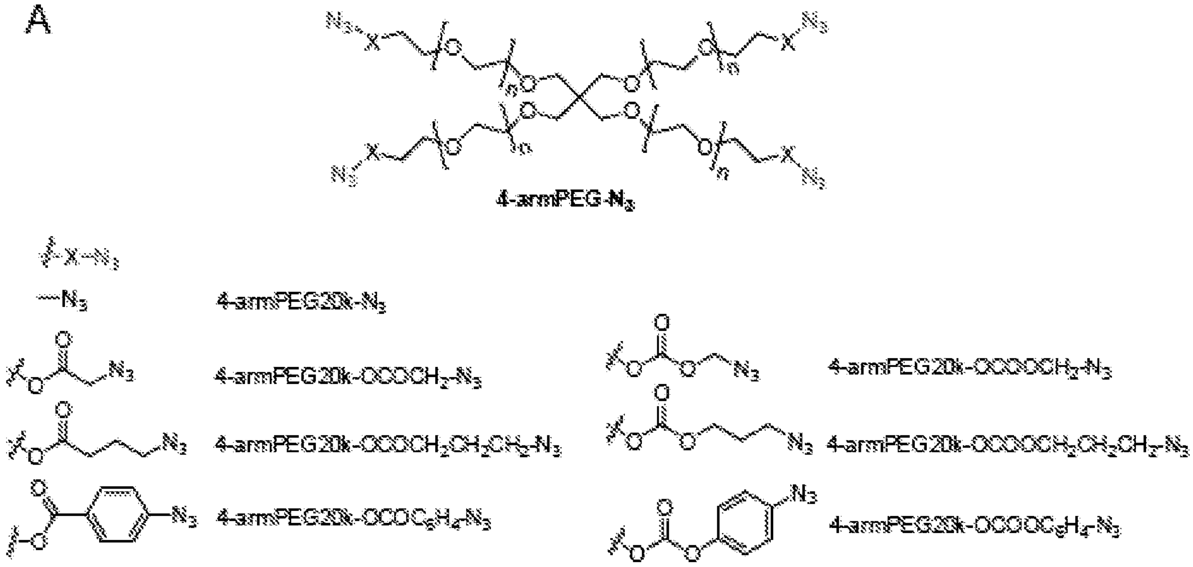

4-armPEG-$N_3$

4-armPEG20k-$N_3$

4-armPEG20k-$OCOCH_2$-$N_3$

4-armPEG20k-$OCOOCH_2$-$N_3$

4-armPEG20k-$OCOCH_2CH_2CH_2$-$N_3$

4-armPEG20k-$OCOOCH_2CH_2CH_2$-$N_3$

4-armPEG20k-$OCOC_6H_4$-$N_3$

4-armPEG20k-$OCOOC_6H_4$-$N_3$

B

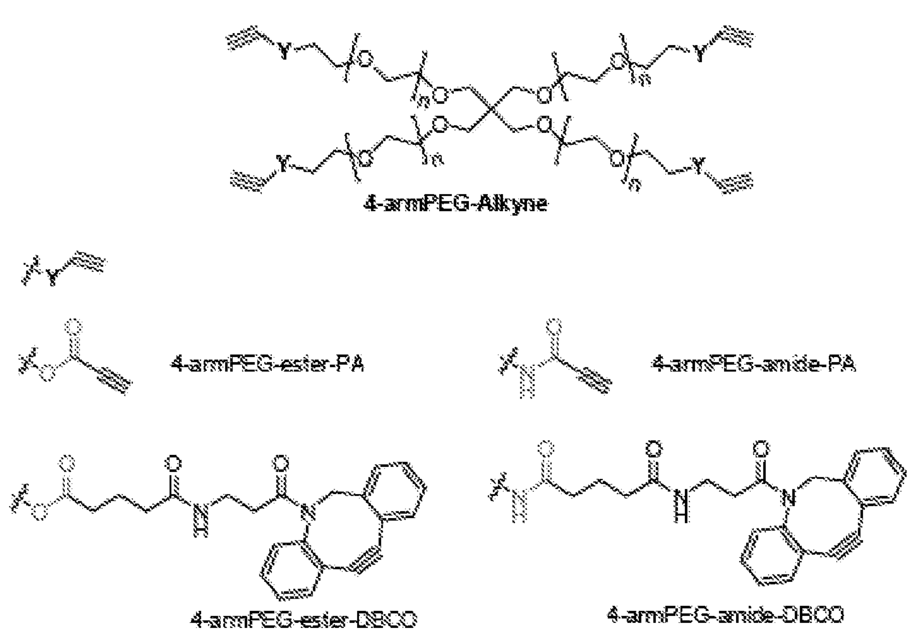

4-armPEG-Alkyne

4-armPEG-ester-PA

4-armPEG-amide-PA

4-armPEG-ester-DBCO

4-armPEG-amide-DBCO

**FIG. 24.** Examples of (A) azido- ($N_3$) and (B) alkyne-terminated macromers containing ester linker with varying lengths/hydrophobicity or reactivity.

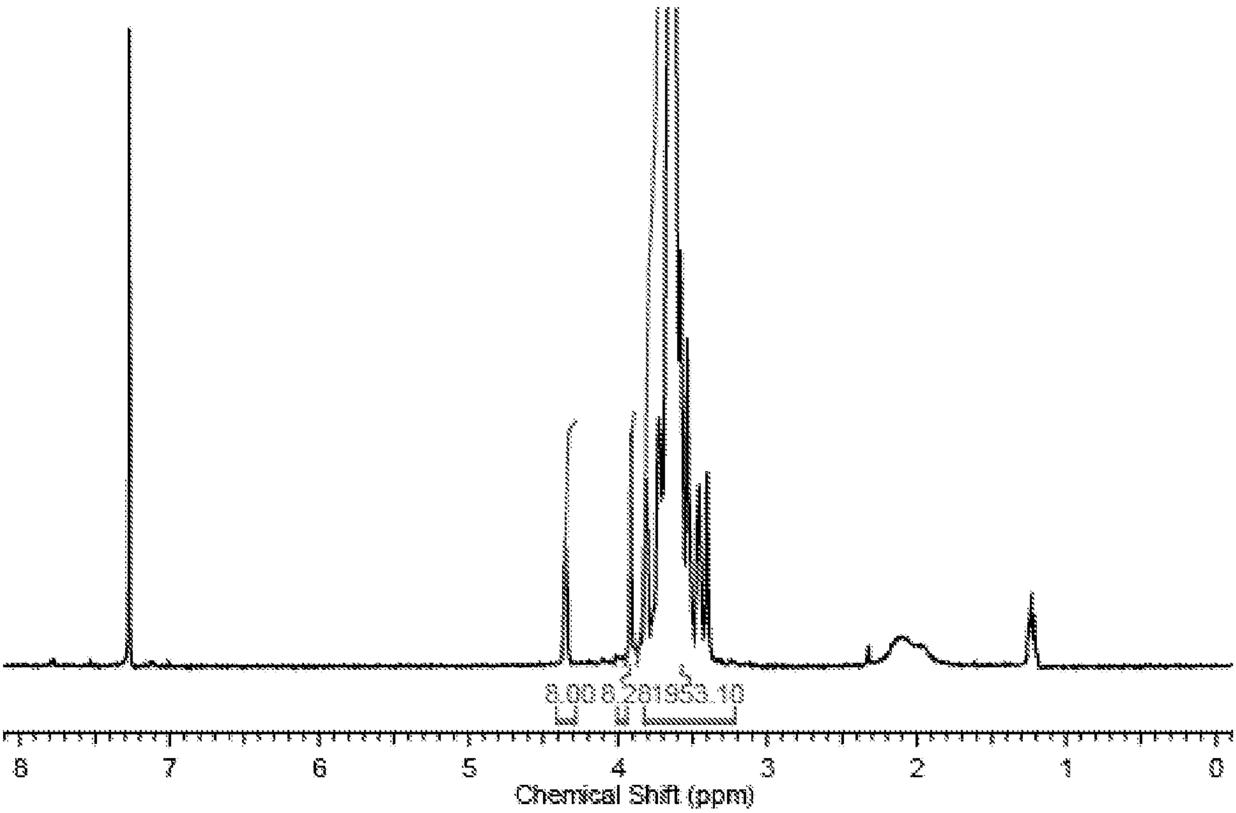


**FIG. 25**. 1H NMR spectra of 4-armPEG-$OCOCH_2N_3$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012116250 A


### Non-patent literature cited in the description


- **ALVAREZ-LORENZO et al.** *J. Drug Deliv. Sci. Tec.,* 2010, vol. 20, 237
- **HOLTZ et al.** *Nature,* 1997, vol. 389, 829
- **DE LANGE et al.** *Acs Applied Materials & Interfaces,* 2011, vol. 3, 50
- **BUHRMAN et al.** *BMC Biotechnology,* 2012, vol. 12, 63
- **KHARKAR et al.** *Chem. Soc. Rev.,* 2013, vol. 42, 7335
- **PEPPAS et al.** *European Journal of Pharmaceutics and Biopharmaceutics,* 2000, vol. 50, 27
- **ZUSTIAK et al.** *Biomacromolecules,* 2010, vol. 11, 1348
- **LI et al.** *Macromolecules,* 2011, vol. 44, 3567
- **GRIFFIN et al.** *J. Am. Chem. Soc.,* 2012, vol. 134, 13103
- **FAIRBANKS et al.** *Macromolecules,* 2011, vol. 44, 2444
- **DEFOREST et al.** *Nature Chemistry,* 2011, vol. 3, 925
- **KLOXIN et al.** *Biomaterials,* 2010, vol. 31, 1
- **DUNN et al.** *J. Am. Chem. Soc.,* 2012, vol. 134, 7423
- **YANG et al.** *Journal of Materials Chemistry B,* 2014, vol. 2, 295
- **LUTOLF et al.** *Proc. Natl. Acad. Sci. U. S. A.,* 2003, vol. 100, 5413
- **EHRBAR et al.** *Biomacromolecules,* 2007, vol. 8, 3000
- **RYDHOLM et al.** *Acta Biomaterialia,* 2007, vol. 3, 449
- **JO et al.** *Soft Matter,* 2009, vol. 5, 440
- **ASHLEY et al.** *Proc. Natl. Acad. Sci. U. S. A.,* 2013, vol. 110, 2318
- **LIN et al.** *Pharmaceutical Research,* 2009, vol. 26, 631
- **NGUYEN et al.** *Biomaterials,* 2012, vol. 33, 6682
- **AGARD et al.** *J. Am. Chem. Soc.,* 2004, vol. 126, 15046
- **XU et al.** *Chemistry-an Asian Journal,* 2011, vol. 6, 2730
- **FLORY.** *Chem. Rev.,* 1946, vol. 39, 137
- **FLORY et al.** *The Journal of Chemical Physics,* 1943, vol. 11, 521
- **MOORE et al.** *Macromolecules,* 1990, vol. 23, 65
- **FRASER et al.** *Medchemcomm,* 2013, vol. 4, 383